# EUROPEAN PATENT APPLICATION

(11) **EP 4 183 797 A1**
(43) Date of publication of application: **24.05.2023**
(21) Application number: 21872754.3
(22) Date of filing: 31.08.2021
(51) Int. Cl.: C07K 14/705, A61K 9/127, A61K 47/42, A61K 47/24, A61P 35/00

(54) **CD47 BINDER AND LIPOSOME COMPLEX FOR TREATING CANCER**

(30) Priority: 22.09.2020 KR 20200122151
(71) Applicant: Bpgene Co., Ltd., Seoul 03127 (KR)
(72) Inventor: LEE, Hyeon Cheol, Seongnam-si, Gyeonggi-do 13510 (KR); KOO, Bong Seong, Seoul 02798 (KR); KIM, Jin Sook, Gwangmyeong-si Gyeonggi-do 14248 (KR); LIM, Jun Sub, Seoul 08602 (KR)
(74) Representative: Swindell & Pearson Limited
(86) International application number: PCT/KR2021/011701
(87) International publication number: WO 2022/065724

(57) **Abstract**

The present invention discloses a binder, which binds to CD47 overexpressed in cancer cells and has an improved binding affinity for CD47, and a liposome complex. The present invention provides a binder, which binds to CD47 overexpressed in cancer cells and includes an amino acid sequence represented by SEQ ID NO: 1 or SEQ ID NO: 2, and a binder-conjugated liposome complex, in which the binder is conjugated to a liposome.

## Description

### TECHNICAL FIELD

The present invention relates to a CD47 binder and a liposome complex, and more specifically, to a binder that binds to CD47 overexpressed in cancer cells and a liposome complex.

### BACKGROUND ART

Cancer cells abuse signals used elsewhere in normal mammalian biochemistry to prevent immune cells from destroying other cells, such as CD47. Interfering with these "don't eat me" signals has produced significant gains in the development of effective cancer therapies that can target multiple types of cancer.

Generally, immune cells called macrophages detect cancer cells and then engulf and devour them. In recent years, researchers have discovered that proteins on the cell surface can send signals to macrophages not to eat or destroy them. These signals are useful to help normal cells keep the immune system from attacking them, but cancer cells also use these "don't eat me" signals to evade the immune system. Researchers have previously shown that the proteins PD-L1 and the beta-2-microglobulin subunit of the major histocompatibility class 1 complex are being used by cancer cells to protect themselves from immune cells. Antibodies that block CD47 are currently in clinical trials and cancer treatments that target PD-L1 or the PDL1 receptors are being used in the treatment of patients.

This study showed that many cancers produce an abundance of CD47 compared to normal cells and surrounding tissues. In recent studies, scientists showed that macrophage cells that infiltrate tumors can sense the CD47 signal through a receptor called Sirpα. They also showed that when cancer cells from patients are mixed with macrophages in a dish and the interaction between CD47 and Sirpα is blocked, the macrophages would start gorging on cancer cells. Lastly, they implanted human breast cancer cells in mice. When CD47 signaling was blocked, the macrophages of the immune system of the mice attacked the cancer. Of particular note was the discovery that blood cancer and triple-negative breast cancer were significantly affected by the blocking of the CD47 signaling.

### [Prior Art Document]

- Korean Patent Application Publication No. 2006-0121150(November 28, 2006)

### DISCLOSURE OF THE INVENTION

### TECHNICAL PROBLEM

An aspect of the present invention provides a binder, which binds to CD47 overexpressed in cancer cells and has an improved binding affinity for CD47, and a liposome complex.

### TECHNICAL SOLUTION

In order to solve the task above, the present invention provides a binder, which binds to CD47 overexpressed in cancer cells and includes an amino acid sequence represented by SEQ ID NO: 1 or SEQ ID NO: 2.

Additionally, the present invention provides the binder which is characterized in that the binder is 1,2-distearoyl-sn-glycero-3-phosphorylethanolamine (DSPE)-conjugated.

Additionally, the present invention provides the binder which is characterized in that the cancer is colorectal cancer or breast cancer.

In order to solve another task above, the present invention provides a binder-conjugated liposome complex in which the binder is conjugated to a liposome.

Additionally, the present invention provides the binder-conjugated liposome complex in which the liposome is positively charged using at least one material selected from the group consisting of cationic phospholipid, DOTAP, and cholesterol.

Additionally, the present invention provides the binder-conjugated liposome complex in which the liposome is PEGylated.

### ADVANTAGEOUS EFFECTS

A binder and a liposome complex according to the present invention have improved binding affinity for CD47; therefore, they can be effectively used as a binder and liposome complex of anticancer agents for treating cancer with high expression of CD47 (e.g., triple negative breast cancer) .

### BRIEF DESCRIPTION OF THE DRAWINGS

FIG. 1 is a diagram illustrating a comparison of the amino acid sequences of Sirpα, SV1, and SV4. Bold letters indicate residues that are not partially conserved. Sequence alignment was performed with ClustalW, and images were generated using the BioEdit sequence alignment editing program.
FIG. 2 is a diagram illustrating the mutation of SV1 for correct orientation. In FIG. 2A, the SV domain (solid box) is complexed with the CD47 (dotted box) domain, and in the model of FIG. 2A, the SV domain (solid box) shows lysine residues affecting correct orientation and that SV4 shows that are mutated by selecting residues not hindered for correct binding to CD47 (underlined). FIG. 2B shows analysis results of simplified DSPE-conjugation through mutation using mass spectrometry analysis of SV1 and SV4.
FIG. 3 is a diagram illustrating the conserved motifs of T001 and human NT5M. As a sequence alignment of T001 and human NT5M, these sequences were aligned using ClustalW to confirm the sequence similarity of human NT5M and T001. Swiss-Prot/TrEMBL accession numbers of the sequences used in the alignment are human NT5M and T001. Inverted fields indicate fully conserved amino acid residues and boxed fields indicate partially conserved amino acid residues with similar biochemical functions. Sequence alignment was made with ClustalW, and images were generated with ESPript server.
FIG. 4 is a model illustrating a comparison of the structures of T001 and NT5M. Cytoplasmic T001 (CT) and dTMP-binding human NT5M are shown as superimposed, and most of the chains are structurally very similar, but the binding loop region does not exist in NT5M but only in CT.
FIG. 5 is a schematic diagram illustrating a candidate structure for UTR screening for optimal expression of T001.
FIG. 6 is images and graphs illustrating the results of FACS analysis in UTR screening for optimal expression of T001. Analysis conditions were as follows: GFP fluorescence, HCT-116, 6-well (5 × 10⁵ cells/well), 24h mRNA transfection, 10% FBS, and 2 mM Gln MEM media.
FIG. 7 is a schematic diagram illustrating N-terminal and C-terminal sequences of mStrawberry. An estimated import signal is located as shown in FIG. 7.
FIG. 8 is images showing the intracellular action positions of mStrawberry-NLS and mStrawberry-MLS revealed by a fluorescence microscope. It can be seen that the positions of the brightly shining fluorescence are each expressed differently depending on the import signal.

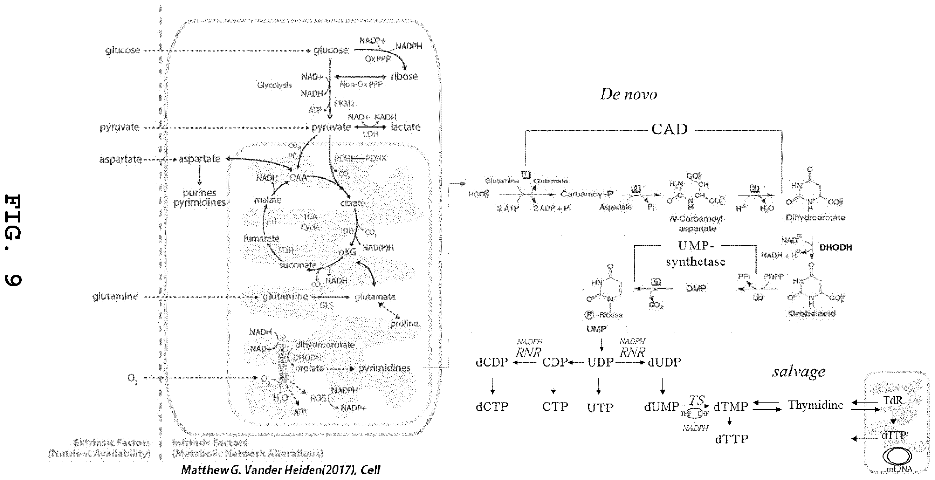

FIG. 9 is a diagram illustrating the mode of action (MOA) and pathway of target metabolism in cancer cells.
FIG. 10 is images showing the results of a live and dead assay after mRNA transfection in MCF7 cell line. The cell viability of MCF7 cells was observed after 5 µg/well of mRNA treatment using a fluorescence microscope. The cell viability at 24 hours after transfection, was effectively reduced, and this effect was either time-dependent or dose-dependent.
FIG. 11 is graphs illustrating the results of MTT analysis after mRNA transfection in MCF7 cell line.
FIG. 12 is graphs illustrating the results of apoptosis analysis according to Annexin V staining after mRNA transfection in MCF7 cell line. The early apoptotic portion (lower right quadrant) was increased continuously after mRNA transfection, and late apoptotic portion (upper right quadrant) was also increased.
FIG. 13 is graphs illustrating results of comparison of cytotoxicity and cell growth inhibition according to T001 transfection and NT5M transfection.
FIG. 14 is graphs illustrating the results of apoptosis induced by CT and NT5M transfection.
FIG. 15 is graphs illustrating a cell cycle arrest by T001 transfection.
FIG. 16 is a graph illustrating a ratio of apoptosis-induced cells according to the concentration in a colorectal cancer cell line HCT-116.
FIG. 17 is graphs illustrating a T001 offset effect by siRNA treatment of T001.
FIG. 18 is graphs illustrating the ratio of apoptosis-induced cells according to a concentration in triple-negative breast cancer (TNBC).
FIG. 19 is images showing the results of Western blot analysis of DNA damage markers after CT treatment for triple-negative breast cancer.
FIG. 20 is a schematic view illustrating an anticancer agent using SV4 binder and T001 drug.
FIG. 21 is a schematic view schematically illustrating the constitution of T001 mRNA.
FIG. 22 is a schematic view and images illustrating the results of an *in vitro* analysis of carboxy fluorescein-DSPE complexed with an immune-liposome (iLP) containing NLS-mStrawberry mRNA. In the MCF7 cell line, the positions of each translated mStrawberry protein and mRNA were confirmed by nuclear transfection through fluorescence detection. FIG. 22A indicates 2.5 µg of NLS-mStrawberry mRNA, and FIG. 22B indicates 5 µg of NLS-mStrawberry mRNA.
FIG. 23 is images showing the results of CD47 masking assay *in vitro* and *in vivo.*
FIG. 24 is images showing the distribution of MCF7 xenograft mice after intravenous (IV) injection of SV4-conjugated iLP-NIR RFP mRNA. FIG. 24A indicates a xenograft mouse, 24B indicates a xenograft mouse 1 hour after injection, 24C indicates a xenograft mouse 3 hours after injection, 24D indicates a xenograft mouse 6 hours after injection, and 24E indicates a cut cancer tissue of a xenograft mouse.
FIG. 25 is a graph and an image illustrating tumor volumes by period after intravenous injection of iLPD *in vivo.*
FIG. 26 is graphs illustrating the results of a toxicity test of mouse organs by iLPD treatment.
FIG. 27 is a schematic diagram illustrating the mechanism of an anticancer agent using the SV4 binder according to the present invention.
FIG. 28 is a diagram illustrating the comparison of DNA sequences of SIRPα and SV1.
FIG. 29 is a diagram illustrating the conjugation process of DSPE-PEG₂₀₀₀-NHS and SV4 proteins.

### MODE FOR CARRYING OUT THE INVENTION

Hereinafter, the present invention will be described in detail through examples. Before going into that, it should be understood that the terms or words used in the present specification and claims should not be construed as being limited to their ordinary or dictionary meanings, but it should be interpreted as meanings and concepts consistent with the technical idea of the present invention based on the principle that the inventors should can properly define the concept of the terms in order to describe the invention in a best mode. Therefore, the constitutions of the examples described in the present specification are merely the most preferred embodiments of the present invention and do not represent all the technical spirit of the present invention, and it should be understood that there may be various equivalents and modifications that can be substituted for them at the time of filing the present application.

The terms used herein may be understood as follows.

The term "CD47" herein is not particularly limited, and may be derived from any animal, preferably a mammal, and more preferably a human CD47. The amino acid sequence and nucleotide sequence of human CD47 are already known (J. Cell. Biol., 123, 485-496, (1993), Journal of Cell Science, 108, 3419-3425, (1995), GenBank: Z25521).

As used herein, the term "binder" refers to a protein that binds to a receptor, in particular, CD47, and the binder binds to CD47, particularly in cancer cells, thereby enabling the recognition and/or interaction of a cell-delivery material.

As used herein, the term "conjugated" or "conjugate" refers to a chemical compound formed by the association of two or more compounds with one or more chemical bonds or linkers. In an embodiment of the present invention, the binder and the liposome form a conjugate.

As used herein, the term "PEGylation" is a technique for increasing stability by conjugating polyethylene glycol (PEG) to a target material. In the present invention, PEGylated phospholipids, for example, DSPE-PEG₂₀₀₀, etc. may be used. DSPE-PEG₂₀₀₀ means DSPE to which PEG having a number average molecular weight of about 2000 is attached.

As used herein, the term "polynucleotide" generally refers to a polymer of deoxyribonucleotides or ribonucleotides present in single-stranded or double-stranded form, which may be RNA or DNA, or modified RNA or DNA. In an embodiment of the present invention, the polynucleotide is synthesized single-stranded mRNA.

As used herein, the term "5'-untransrated region (5'-UTR)" is commonly understood as a specific portion of mRNA located 5' of the protein coding region (i.e., open reading frame (ORF)) of the mRNA. Typically, the 5'-UTR starts at the transcription start region and ends one nucleotide before the start codon of the open reading frame.

As used herein, the term "3'-Untransrated Region (3'-UTR)" is a section of mRNA that is usually located between the open reading frame (ORF) of the mRNA and a poly (A) sequence. The 3'-UTR of mRNA is not translated into an amino acid sequence. The 3'-UTR sequence is usually encoded by a gene that is transcribed into each mRNA during gene expression.

As used herein, the terms "nuclear localization signal (NLS)" and "mitochondrial localization signal (MLS)" each refer to an amino acid sequence that serves to transport a specific material (*e*.*g*., protein or nucleic acid) into the cell nucleus and mitochondria.

As used herein, the term "transfection" refers to a process in which an extracellular polynucleotide enters a host cell, in particular, a cancer cell, in a state with or without accompanying materials. A "transfected cell" may refer to, for example, a cell in which an extracellular mRNA is introduced into the cell and thus has extracellular mRNA.

The present invention discloses a binder that binds to CD47 overexpressed in cancer cells and includes an amino acid sequence represented by SEQ ID NO: 1 or SEQ ID NO: 2.

In the present invention, the binder including the amino acid sequence represented by SEQ ID NO: 1 was named 'SV1', and the binder including the amino acid sequence represented by SEQ ID NO: 2 was named 'SV4'.

First, Sirpα-variant-version 1 (SV1) was selected from a mutant library derived from pure Sirpα (amino acid sequence (SEQ ID NO: 25)), which is the water-soluble domain of the original CD47 ligand. The selection process for SV1 mutants is as follows.

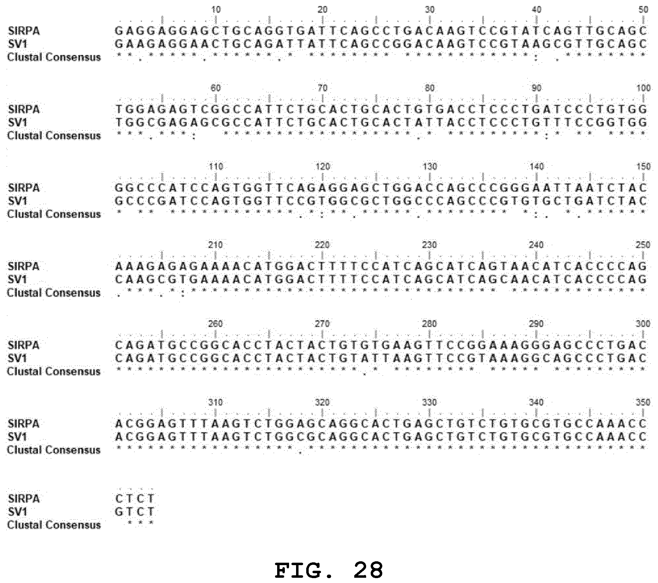

A single 14-kD binding domain of human SIRPα was synthesized to secure the gene by referring to proteins with improved binding affinity of SIRPα binding to CD47 Weiskopf K et *al.* (Weiskopf, K., et al. (2013). "Engineered SIRPalpha variants as immunotherapeutic adjuvants to anticancer antibodies." Science (New York, NY) 341) (6141): 88-91.). The amino acids changed from wild-type SIRPα are as follows. The valine at the 6th position was substituted with isoleucine, the valine at the 27th position with isoleucine, the isoleucine at the 31st position with phenylalanine, and the glutamate at the 47th position with valine, the lysine at the 53rd position with arginine, the glutamate at the 54th position with glutamine, the histidine at the 56th position with proline, the serine at the 66th position with threonine, and the valine at the 92nd position with isoleucine. For the expression of the protein in *E. coli,* SV1 was prepared by modifying an existing nucleic acid sequence through codon optimization, and the SIRPα sequence (SEQ ID NO: 26) and the DNA sequence of SV1 (SEQ ID NO: 27) were compared and the results are shown in FIG. 28.

Meanwhile, SV1-(C)CRM197, which is a protein in which CRM197 protein is added to the C-terminus of SV1, CRM197(N)-SV1 in which CRM197 protein is added to the N-terminus, and SV1 protein were prepared, and the effects of the addition of these proteins at the end were confirmed through surface plasmon resonance (SPR) analysis. Each sample was analyzed through the binding kinetics of SPR using a Biacore X100 instrument. In this case, as the chip for analysis, a chip in which the recombinant CD47 protein is conjugated to the Protein G surface to 500 RU was used, and HBS-P was analyzed at a flow rate of 5 µL/minutes. The results of each sensogram were analyzed with the BiaEvaluation software using a 1:1 binding model and global fitting, and the results are shown in Table 1 below.

**[Table 1]**

| Analyte | K_{D} (nM) |
|---|---|
| SV1 protein | 0.877 |
| SV1-CRM197 protein | 0.894 |
| CRM197-SV1 protein | 19.9 |

Referring to Table 1, reviewing the SPR analysis results, it was confirmed that while SV1-CRM197 with CRM197 inserted at the C-terminus was not changed significantly in K_{D} value compared to SV1, CRM197-SV1 with CRM197 inserted at the N-terminus showed about a 2-fold increase in the K_{D} value to 19.9 nM, thereby reducing the affinity. Therefore, it was confirmed that when a protein is added at the N-terminus of SV1, the affinity of SV for CD47 is decreased compared to that of SV1, due to the steric hindrance that occurs by preventing SV from correctly binding to CD47.

Hereinafter, a SV4 binder according to the present invention, a liposome conjugated with the binder, a T001 drug captured in the binder-conjugated liposome, the SV4 binder and an anticancer agent using the T001 drug will be described in detail with reference to specific examples of the present invention.

### SV4-conjugated liposome

A drug delivery system based on lipid nanoparticles has been universally used due to the significant advantages. Lipid nanoparticles have high drug capacity, high stability, and high specificity, and the release point can be controlled. Due to genetic materials used as drugs, a cationic liposome was used to deliver drugs to target cancers. A positively charged liposome draws in the genetic drug and forms a spherical complex covering up the drug for protection until the drug meets the target.

In the present invention, in order to form cationic liposome, pro-liposome was prepared using one of the cationic phospholipids, DOTAP, and cholesterol. Additionally, for the increase of serum stability during the delivery of the drug to the target through blood vessels, PEG₁₀₀₀-DSPE was additionally incorporated into the pro-liposome to prepare PEGylated liposome. Besides of the PEGylated liposome, DSPE-PEG₂₀₀₀-SV4 was prepared by conjugating NHS-activated DSPE-PEG₂₀₀₀ with SV4 protein. As a final step to prepare the SV4 conjugated liposome, mRNA encapsulated pro-liposome was mixed with DSPE-PEG₂₀₀₀-SV4. The specific preparation process of SV4-conjugate liposome is as follows.

Liposome was prepared in a dry-film manner. Cationic liposome consisting of DOTAP (Avanti Polar Lipids) and cholesterol (Sigma) (1:1 molar ratio, 10 mM) and PEG-DSPE1000 (1 mM; Avanti Polar Lipids) were added. The cationic liposome was dissolved in chloroform and methanol (2:1 (v/v)) in a round bottom glass flask. Lipids were dried under vacuum with a rotary evaporator at 50°C. In order to completely remove chloroform and methanol, a lipid membrane was freeze-dried overnight. After evaporation, the lipid membrane was rehydrated with nuclease-free water at 50°C for up to 1 hour. The hydrated lipid membrane was sonicated to form unilamellar vesicles. Finally, the lipids were extruded using a mini-extruder (Avanti Polar Lipids) using a membrane with 100 nm pores.

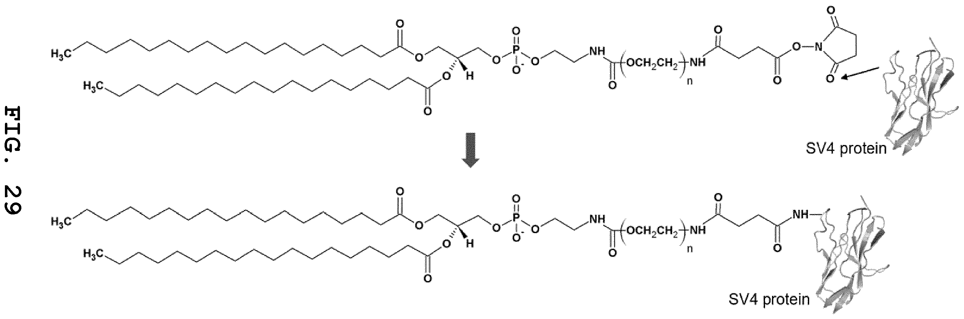

The conjugation of DSPE-PEG₂₀₀₀-NHS and SV4 protein was prepared as follows (see FIG. 29). The DSPE-PEG₂₀₀₀-NHS was prepared in a dry-film method. The DSPE-PEG₂₀₀₀-NHS dissolved in chloroform was evaporated in a round bottom glass flask. Lipid drying was performed by a rotary evaporator at 30°C under vacuum for 1 hour. After evaporation, the lipid membrane was rehydrated with SV4 protein dissolved in nuclease-free water at 30°C for 1 hour. In order to remove residual unconjugated DSPE-PEG₂₀₀₀-NHS, dialysis was performed using a dialysis cassette 10,000 MWCO (Thermo Scientific) overnight in PBS at pH 6.8.

Liposome containing an encapsulated drug and a binding ligand was prepared as described above. Cationic liposome 3 mg, protamine 25 µg, and diethylpyrocarbonate water were mixed to prepare solution A, and mRNA 50 µg and diethylpyrocarbonate water were mixed to prepare solution B. The solutions A and B were incubated for 30 minutes by equalizing the volume with diethyl pyrocarbonate water. Thereafter, the liposome was formed with the encapsulated drug by mixing and incubating for 30 minutes. For the binding of DSPE-PEG₂₀₀₀-NHS conjugated SV4 ligand, liposome containing the encapsulated drug (1:100 molar ratio) were mixed at 50°C for 15 minutes.

### SV1 as Conjugation Target

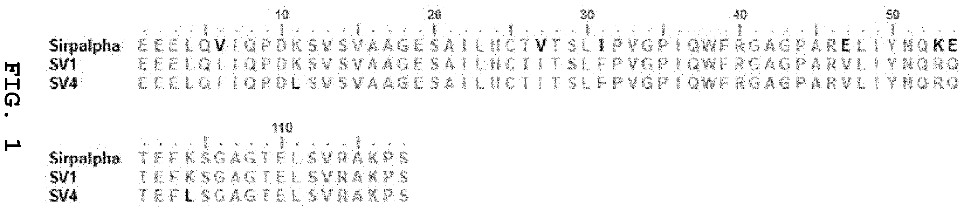

SV1, which has improved binding affinity for CD47 by modifying the sequence of Sirpα in nature, was selected through mutation, and SV4, which was inserted in the correct orientation to be reacted when preparing a liposome formulation, was secured through mutation (see FIG. 1). A preparation of an SV4 mutant was performed by the following method. Lysine residues at the 11th and 104th positions in a secured SV1 sequence were substituted with leucine for the binding in the correct orientation with CD47. For substitution, primers were prepared using a plasmid in which the SV1 gene was inserted into the pET28a vector, such that the sequences corresponding to the 11th and 104th positions are point-mutated (see Table 2 below) and Quickchange II site-directed mutant genes substituted individually or simultaneously were prepared according to the method of the mutagenesis kit (Agilent).

**[Table 2]**

| Category | | Sequence | SEQ ID NO |
|---|---|---|---|
| Leu 11 mutagene sis | (KKtoLL11_F1) Forward | GAT TAT TCA GCC GGA CCT GTC CGT AAG CGT TGC | 28 |
| | (KKtoLL11_R1) Reverse | GCA ACG CTT ACG GAC AGG TCC GGC TGA ATA ATC | 29 |
| Leu 104 mutagene | (KKtoLL104_F2) Forward | CTG ACA CGG AGT TTC TGT CTG GCG CAG | 30 |
| sis | (KKtoLL104_R2) Reverse | CTG CGC CAG ACA GAA ACT CCG TGT CAG | 31 |

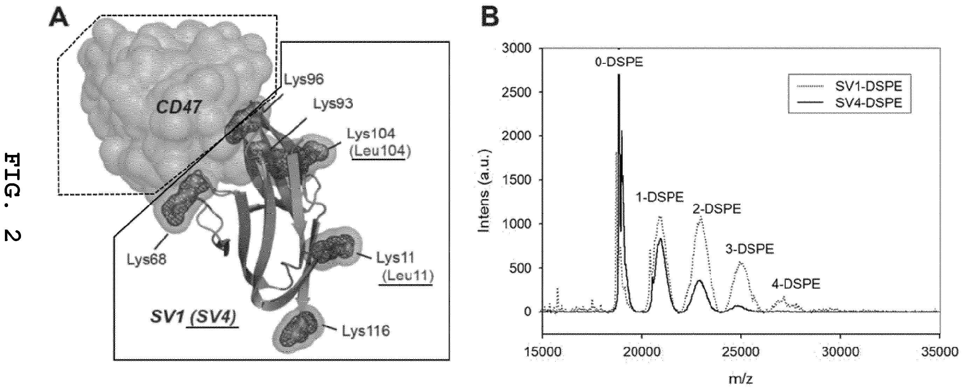

SV1 protein has six lysine residues in addition to the N-terminal amino group (see FIG. 2A). In order to link SV1 to a liposome through conjugation to deliver a drug through the binding to CD47, it is necessary to improve the binding affinity. Through NHS conjugation, it is possible to select residues that can function in the correct orientation without interfering with the binding to CD47 among the residues that can act in the chemical reaction linking DSPE and modify without loss of binding activity by substituting residues other than these residues with leucine. As shown in FIG. 2, DSPE-conjugated SV4 was prepared through simple binding by reducing the number of residues binding to DSPE through substitution.

Meanwhile, in order to confirm the effect of lysine substitution for the correct orientation of SV4, a DSPE-conjugated form and a liposome-inserted form were each prepared for comparison with SV1, and the affinity with CD47 was analyzed by surface plasmon resonance (SPR). For analysis, after conjugation of CD47 using a CM5 chip, the association and dissociation of SV1, DSPE-SV1, LPD-SV1, SV4, DSPE-SV4, and LPD-SV4 were measured and the K_{D} values were compared. Specifically, the recombinant CD47 protein was conjugated to the surface of the CM5 chip by a target SPR reaction of 250 RU through an EDC-NHS reaction to be used, and for HBS-P, the sensogram obtained through a process of association for 3 minutes and dissociation for 10 minutes at a flow rate of 30 µL/minutes was analyzed with the BiaEvaluation software using a 1:1 binding model and global fitting. According to the characteristics of the analysis sample, 10 mM glycine at pH 2.0 was used as a dissociation buffer for SV1 and SV4 proteins, and 2.0 M MgCl₂ was used for the sample to which fatty acids were attached.

As a result of SPR analysis, SV1 showed a significant improvement in the K_{D} value from 280 nM to 0.87 nM compared to Sirpα(wt) (see Table 3). Although the affinity of SV1 was greatly improved, when SV1-DSPE was prepared through the NHS conjugation reaction, the affinity was decreased due to the increase of the K_{D} value from 0.87 nM to 2.67 nM. Additionally, in the case of SV1-iLP inserted into the liposome, the affinity was further decreased, and the K_{D} value increased to 10.9 nM. In contrast, in the case of SV4, in which two lysines which are NHS reaction residues were substituted with leucine, it was confirmed that the affinity of the protein itself decreased slightly, but due to the correct orientation, the binding activity was maintained within the error range without a significant decrease when conjugated with DSPE and even when inserted into liposomes. (see Table 3).

**[Table 3]**

| Analyte | *kₐ* (M⁻¹s⁻¹) | *k_{d}* (s⁻¹) | K_{D} (nM) |
|---|---|---|---|
| Sirpα | 6.10×10⁵ | 3.70×10⁻¹ | 290.0 |
| SV1 | 3.53×10⁵ | 3.08×10⁻⁴ | 0.87 |
| SV1-DSPE | 7.34×10⁴ | 1.96×10⁻⁴ | 2.67 |
| SV1-iLP | 1.73×10⁴ | 1.89×10⁻⁴ | 10.9 |
| SV4 | 1.64×10⁵ | 2.75×10⁻⁴ | 1.67 |
| SV4-DSPE | 2.27E×10⁵ | 2.56×10⁻⁴ | 1.13 |
| SV4-iLP | 3.12E×10⁵ | 3.95×10⁻⁴ | 1.27 |

### Binder-Conjugated Liposome Capturing Drug

Hereinafter, embodiments of the above-described binder (SV4)-conjugated drug captured in the liposome will be described.

Thymidylate 5'-phosphohydrolase derived from bacteriophage PBS2 (PBS2 phage), which has activity similar to human 5'-nucleotidase, but has exceptionally high specificity only for dTMP and dUMP, while having no specificity for other nucleic acids, was selected as a drug candidate material having a mechanism to kill cancer cells by maximizing the metabolic vulnerability of cancer cells; and the gene sequence was optimized such that the site that acts as a non-specific microRNA (microRNA) when delivered into human cells is minimized while the expression is maximized, and named 'T001' (SEQ ID NO: 3). The sequence optimization process of T001 is as follows.

In order to optimize the DNA sequence for mammalian cell expression, natural codons were replaced with the following optimal codons: alanine (GCC), arginine (CGC), asparagine (AAC), aspartic acid (GAC), cysteine (TGC), glutamic acid (GAG), glutamine (CAG), glycine (GGC), histidine (CAC), isoleucine (ATC), leucine (CTG), lysine (AAG), methionine (ATG), phenylalanine (TTC), proline (CCC), serine (TCC), threonine (ACC), tryptophan (TGG), tyrosine (TAC), and valine (GTG). Runs of Cs and Gs were avoided to simplify PCR conditions as well as oligonucleotide synthesis.

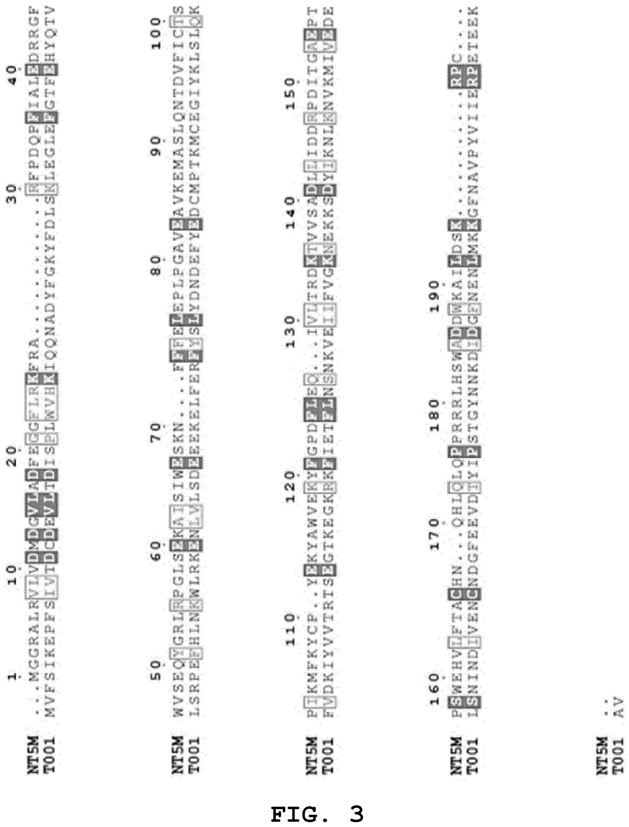

The enzyme involved in nucleic acid metabolism in human cells similar to T001 is 5'-nucleotidase, and according to the site of action, three types are known: NT5C (cytoplasm), NT5M (mitochondria), and NT5E (extracellular membrane). These three enzymes differ in their preferred substrate nucleic acid species due to differences in hydrolyzing activity and structure of the phosphate group of NMP or dNMP as well as the site of action, and most of them are known to have broad specificity for NMP or dNMP. Among them, NT5M (see SEQ ID NO: 32) was estimated to be generally similar in structure to T001 despite the low similarity in the amino acid sequence compared to T001 (see FIG. 3). In particular, it was confirmed that the sequence for the action site was conserved despite the sequence difference, and that it belongs to the haloalkanoic acid dehalogenase (HAD) superfamily.

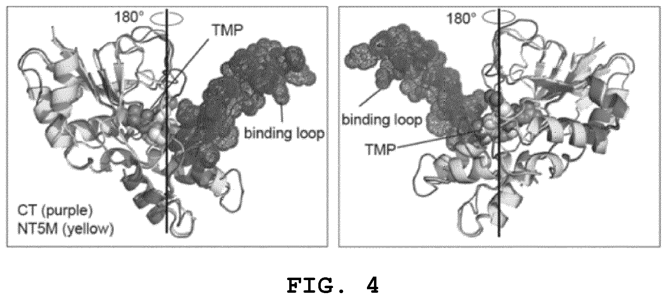

Despite the structural homology, the structural difference between NT5M and T001 lies in that T001 has a unique binding loop structure that does not exist in NT5M, and this is the biggest characteristic difference (see FIG. 4) .

The binding loop is closely related to the substrate binding sites of NT5M and T001, and is presumed to be related to the specificity of the substrate. Although little is known about the function of the binding loop until now, it was partially confirmed in the present invention that, due to the binding loop, T001 has higher affinity for dTMP and high dTMP resolution compared to NT5M (see Table 4).

**[Table 4]**

| 5' NT | Alias | Preferred Substrate (Km) | References |
|---|---|---|---|
| PBS2 TMP phosphohydrolase | T001 | | *Methods Enzymol.* |
| | | dTMP (0.01 mM) | 51:285-290 |
| | | dGMP (0.7 mM) | (1978), |
| | | dUMP (0.8 mM) | Also In house data |
| Human mitochondrial 5'doxyribonucleo tidase | NT5M (hdNT-2) | | *Biochem.* |
| | | dGMP (0.09 mM) | 46:13809-13818 |
| | | dUMP (0.16 mM) | (2007) |
| | | dTMP (0.3 mM) | Biochemical Pharmacol. 66: 471-479 (2003) |
| Human cytosolic 5'deoxyribonucle otidase | hdNT-1 | dUMP (1.5 mM) | *J*. *Biol. Chem.* |
| | | | 265:6589-6595 |
| | | dTMP (1.5 mM) | (1990) |
| | | dAMP (3.0 mM) | Biochemical Pharmacol. 66: |
| | | dGMP (3.3 mM) | |
| | | | 471-479 (2003) |
| Murine cytosolic 5'deoxyribnucleo tidase | mdNT-1 | dUMP (0.8 mM) | *J. Biol. Chem.* |
| | | | 275:5409-5415 |
| | | dAMP (1.0mM) | (1990) |
| | | dGMP (1.2mM) | *Biochemical* |
| | | dTMP (1.4 mM) | Pharmacol. 66: 471-479 (2003) |

As shown in Table 4, it is known that the affinity for dTMP is the highest among other 5'-deoxyribonucleotidases known to date. According to what have been reported thus far, it can be seen that other enzymes exhibit relatively broad substrate specificity as well as lower affinity for dTMP compared to T001 presented in the present invention. In particular, even when compared to structurally similar human NT5M, the spectrum for the substrate is different and the affinity for dTMP is also 30 times lower according to Table 4. This difference is presumed to be due to the presence of a binding loop in T001 that does not exist in NT5M.

### T001 mRNA drug - localization and mRNA structure

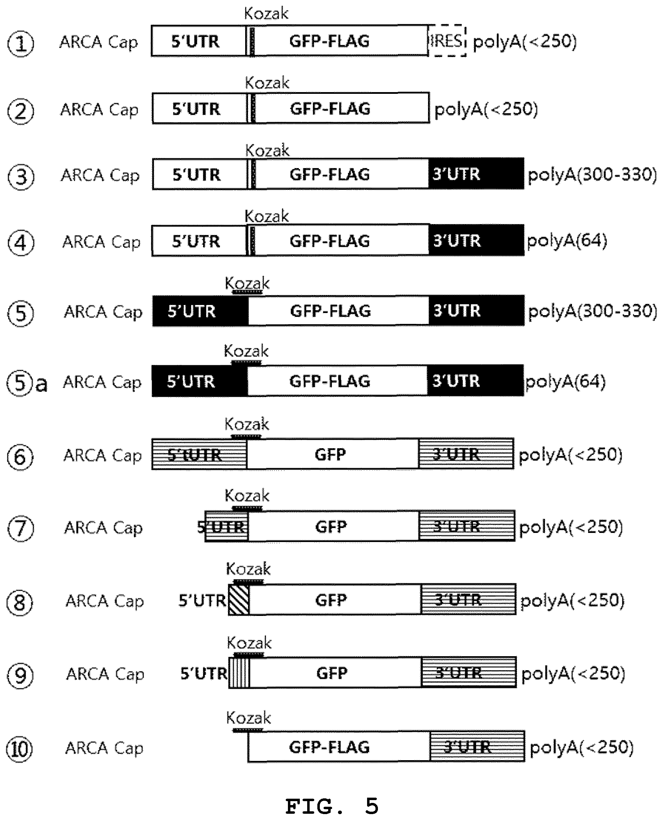

The form of the final drug of T001 was an mRNA form, and untranslated region (UTR) and Kozak sequences were optimized by optimizing each component required for expression. For this, the expression efficiency was determined by selecting a candidate structure as shown in FIG. 5 using green fluorescent protein (GFP) as a reporter gene. The sequence information of each UTR used is shown in Table 5 below.

**[Table 5]**

| N O | 5'-UTR | 5'-UTR source | 3'-UTR | 3 v - UTR source | Ref |
|---|---|---|---|---|---|
| 1 | ttggtcgtgaggcact gggcaggtaagtatca aggttacaagacaggt ttaaggagaccaatag aaactgggcttgtcga gacagagaagactctt gcgtttctgataggca cctattggtcttactg acatccactttgcctt tctctccacaggtgtc cactcccagttcaatt a (SEQ ID NO: 4) | Chimeric intron | GAATTCACGCGTCGAGCATG CATCTAGGGCGGCCAATTCC GCCCCTCTCCCCCCCCCCCC TCTCCCTCCCCCCCCCCTAA CGTTACTGGCCGAAGCCGCT TGGAATAAGGCCGGTGTGCG TTTGTCTATATGTTATTTTC CACCATATTGCCGTCTTTTG GCAATGTGAGGGCCCGGAAA CCTGGCCCTGTCTTCTTGAC GAGCATTCCTAGGGGTCTTT CCCCTCTCGCCAAAGGAATG CAAGGTCTGTTGAATGTCG (SEQ ID NO: 5) | IRES | - |
| 2 | ttggtcgtgaggcact gggcaggtaagtatca aggttacaagacaggt ttaaggagaccaatag aaactgggcttgtcga gacagagaagactctt gcgtttctgataggca cctattggtcttactg acatccactttgcctt tctctccacaggtgtc cactcccagttcaatt a(SEQ ID NO: 6) | Chimeric intron | - | | - |
| 3 | ttggtcgtgaggcact gggcaggtaagtatca aggttacaagacaggt ttaaggagaccaatag aaactgggcttgtcga gacagagaagactctt gcgtttctgataggca cctattggtcttactg acatccactttgcctt tctctccacaggtgtc cactcccagttcaatt a(SEQ ID NO: 7) | Chimeric intron | GACTAGTGCATCACATTTAA AAGCATCTCAGCCTACCATG AGAATAAGAGAAAGAAAATG AAGATCAATAGCTTATTCAT CTCTTTTTCTTTTTCGTTGG TGTAAAGCCAACACCCTGTC TAAAAAACATAAATTTCTTT AATCATTTTGCCTCTTTTCT CTGTGCTTCAATTAATAAAA AATGGAAAGAACCTAGATCT AAAAAAAAAAAAAAAAAAAA AAAAAAAAAAAAAAAAAAAA AAAAAAAAAAAAAAAAAAAA AAAATGCATCCCCCCCCCCC CCCCCCCCCCCCCCCCCCCC AAAGGCTCTTTTCAGAGCCA CCAGAATT (SEQ ID NO: 8) | Album in | And reas The ss( 201 5) |
| 4 | ttggtcgtgaggcact gggcaggtaagtatca aggttacaagacaggt ttaaggagaccaatag aaactgggcttgtcga gacagagaagactctt gcgtttctgataggca cctattggtcttactg acatccactttgcctt tctctccacaggtgtc cactcccagttcaatt a(SEQ ID NO: 9) | Chimeric intron | GACTAGTGCATCACATTTAA AAGCATCTCAGCCTACCATG AGAATAAGAGAAAGAAAATG AAGATCAATAGCTTATTCAT CTCTTTTTCTTTTTCGTTGG TGTAAAGCCAACACCCTGTC TAAAAAACATAAATTTCTTT AATCATTTTGCCTCTTTTCT CTGTGCTTCAATTAATAAAA AATGGAAAGAACCTAGATCT AAAAAAAAAAAAAAAAAAAA AAAAAAAAAAAAAAAAAAAA AAAAAAAAAAAAAAAAAAAA AAAATGCATCCCCCCCCCCC CCCCCCCCCCCCCCCCCCCC AAAGGCTCTTTTCAGAGCCA CCAGAATT (SEQ ID NO: 10) | Album in | And reas The ss( 201 5) |
| 5 | GGGTCCCGCAGTCGGC GTCCAGCGGCTCTGCT TGTTCGTGTGTGTGTC GTTGCAGGCCTTATTC AAGCTTGAGG(SEQ ID NO: 11) | Andreas Thess(2 015) | GACTAGTGCATCACATTTAA AAGCATCTCAGCCTACCATG AGAATAAGAGAAAGAAAATG AAGATCAATAGCTTATTCAT CTCTTTTTCTTTTTCGTTGG TGTAAAGCCAACACCCTGTC TAAAAAACATAAATTTCTTT AATCATTTTGCCTCTTTTCT CTGTGCTTCAATTAATAAAA AATGGAAAGAACCTAGATCT AAAAAAAAAAAAAAAAAAAA AAAAAAAAAAAAAAAAAAAA AAAAAAAAAAAAAAAAAAAA AAAATGCATCCCCCCCCCCC CCCCCCCCCCCCCCCCCCCC AAAGGCTCTTTTCAGAGCCA CCAGAATT (SEQ ID NO: 12) | Album in | And reas The ss( 201 5) |
| 6 | CTTCCTACTCAGGCTT TATACAAAGACCAAGA GGTACAGGTGCAAGGG AGAGAAGAAGAGTAAG AAGAAATATAAGAGCC ACC(SEQ ID NO: 13) | Musmusculus_alpha -globin (Hbat1) | TTAATTAAGCTGCCTTCTGC GGGGCTTGCCTTCTGGCCAT GCCCTTCTTCTCTCCCTTGC ACCTGTACCTCTTGGTCTTT GAATAAAGCCTGAGTAGGAA G (SEQ ID NO: 14) | Musmusculus hemoglobin alpha (Hba-a1) | UTR db |
| 7 | AGTAAGAAGAAATATA AGAGCCACC(SEQ ID NO: 15) | Rhinatrema bivitta turn | TTAATTAAGCTGCCTTCTGC GGGGCTTGCCTTCTGGCCAT GCCCTTCTTCTCTCCCTTGC ACCTGTACCTCTTGGTCTTT | Musmusculus hemog | UTR db |
| | | thiamin pyropho sphokin ase 1 | GAATAAAGCCTGAGTAGGAA G (SEQ ID NO: 16) | lobin alpha (Hba-a1) | |
| 8 | gggagactgccacc(S EQ ID NO: 17) | Željka Trepote c(2019) | TTAATTAAGCTGCCTTCTGC GGGGCTTGCCTTCTGGCCAT GCCCTTCTTCTCTCCCTTGC ACCTGTACCTCTTGGTCTTT GAATAAAGCCTGAGTAGGAA G (SEQ ID NO: 18) | Musmusculus hemoglobin alpha (Hba-a1) | UTR db |
| 9 | gggagactgccaag(S EQ ID NO: 19) | Željka Trepote c(2019) | TTAATTAAGCTGCCTTCTGC GGGGCTTGCCTTCTGGCCAT GCCCTTCTTCTCTCCCTTGC ACCTGTACCTCTTGGTCTTT GAATAAAGCCTGAGTAGGAA G (SEQ ID NO: 20) | Musmusculus hemoglobin alpha (Hba-a1) | UTR db |
| 1 0 | GCTAGC (SEQ ID NO: 21) | NheI | TTAATTAAGCTGCCTTCTGC GGGGCTTGCCTTCTGGCCAT GCCCTTCTTCTCTCCCTTGC ACCTGTACCTCTTGGTCTTT GAATAAAGCCTGAGTAGGAA G (SEQ ID NO: 22) | Musmusculus hemoglobin alpha (Hba-a1) | UTR db |

Expression efficiency for each UTR was analyzed by FACS through the GFP fluorescence level. The FACS analysis method is as follows.

5 × 10⁵ Cells were placed in each well of a 6 cell culture plate, incubated for 24 hours in a cell incubator at 37°C and 5% CO₂ conditions to be attached to the bottom of the plate, and then EGFP mRNA expressing green fluorescence having each UTR structure was transfected using lipofectamine messengerMAX reagent. First, 125 µL of OPTI-MEM medium and 3.5 µL of the reagent were mixed in a micro-tube and incubated at room temperature for 10 minutes. In another micro-tube, an mRNA dilution medium, in which 125 µL of medium and 1.25 µg of mRNA having each UTR structure were added and mixed, was added to the reagent above and mixed. After incubating for additional 5 minutes, the resultant was administered to the cells of each well. After 4 hours, the cells of each well were lightly washed with phosphate buffered saline, replaced with a cell culture medium, and cultured for additional 20 hours. The expression levels of green fluorescent protein of EGFP mRNA of each UTR were compared and analyzed by fluorescence microscopy and flow cytometry. As a control group, EGFP mRNA purchased from Trilink Biotechnology was used. First, the intensity of green fluorescence of the cells after incubation was compared and analyzed as an image through a fluorescence microscope. Then, the cells treated with EGFP mRNA having each UTR including the control were detached from the plate bottom with trypsin enzyme, harvested in a microcentrifuge tube, and then diluted in phosphate buffered saline. With regard to the thus-harvested cells, the distribution of the cell group expressing green fluorescence was compared to the control group in three stages of strong, medium, and weak according to the intensity through a flow cytometer.

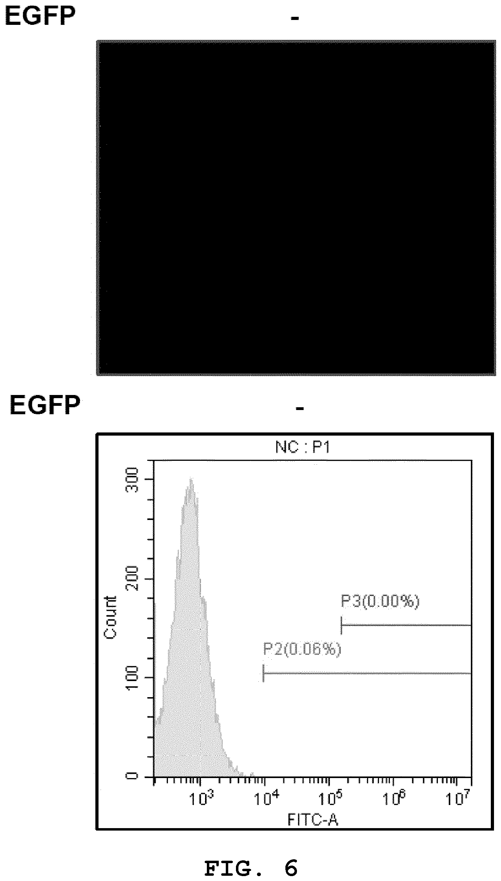

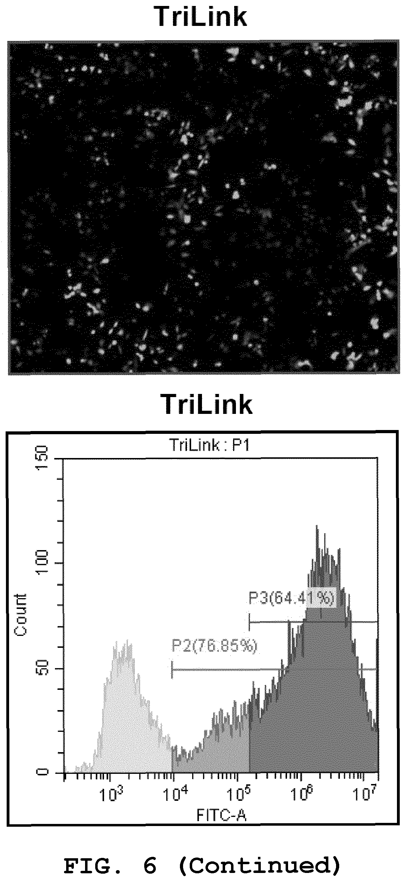

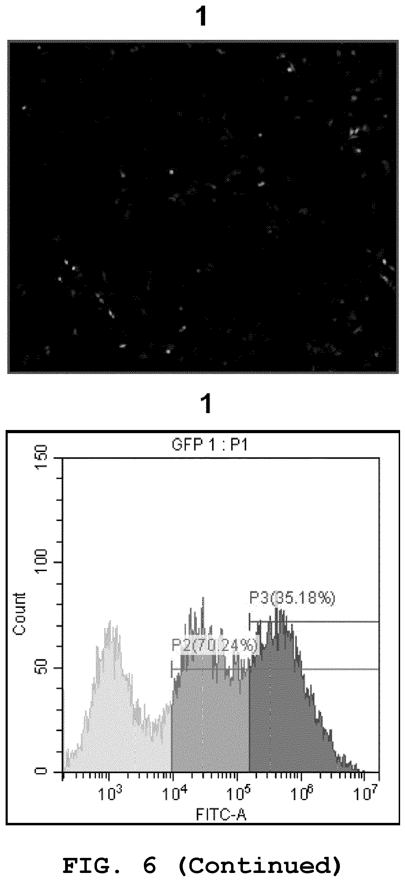

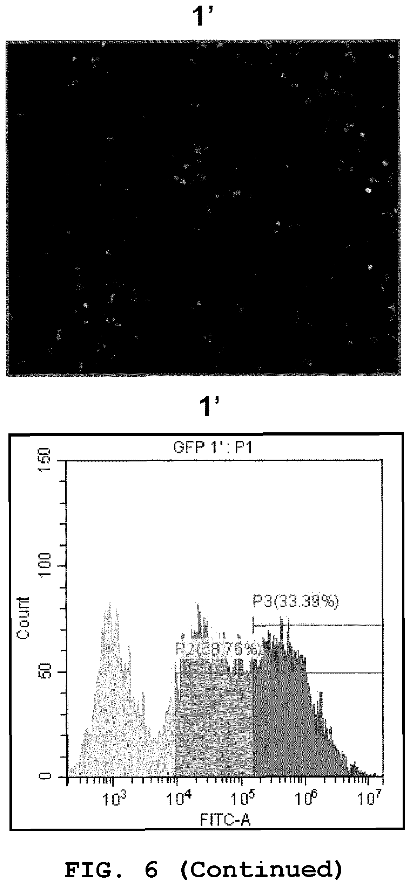

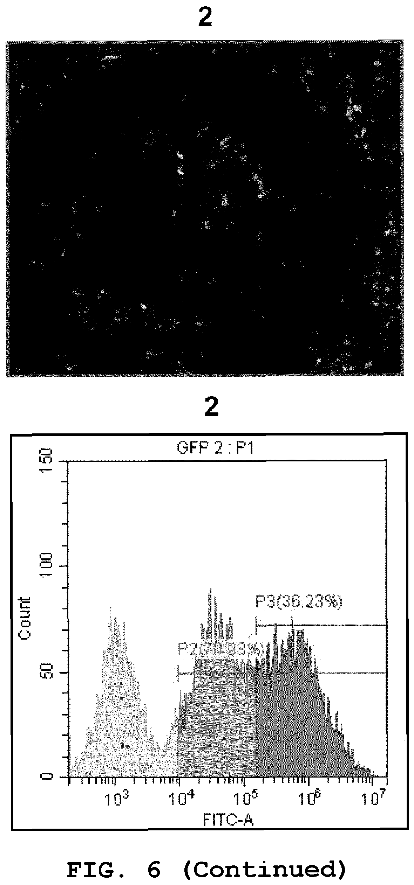

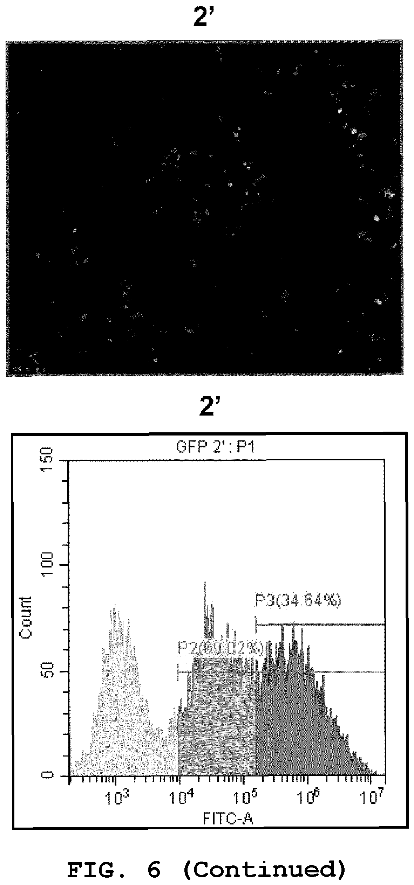

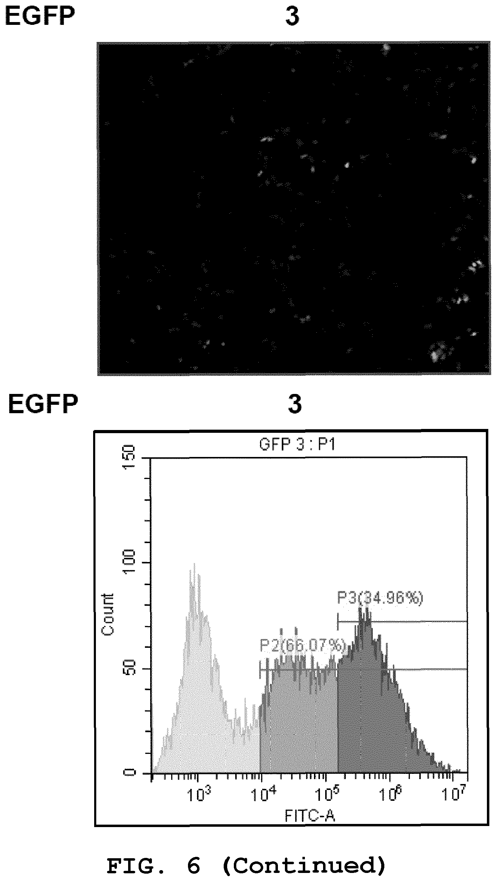

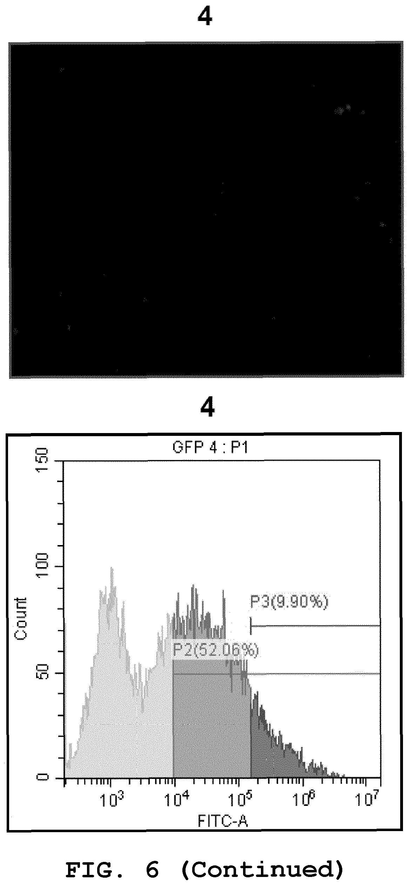

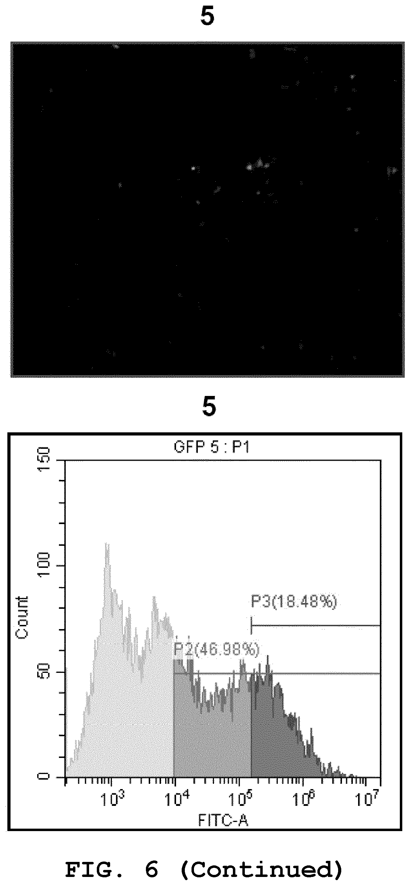

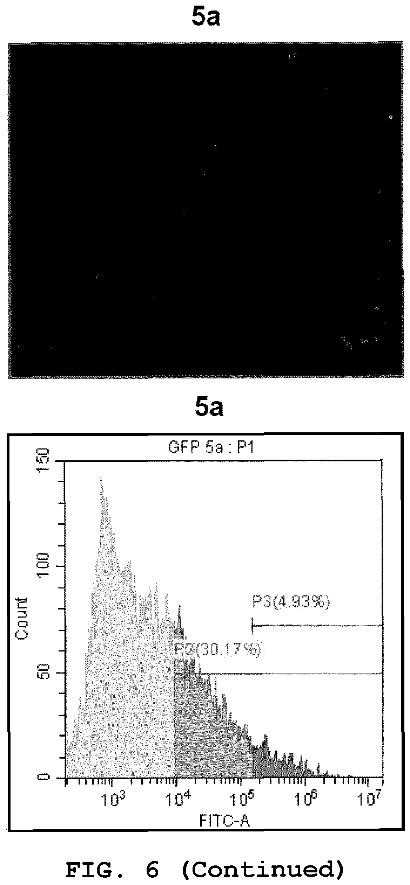

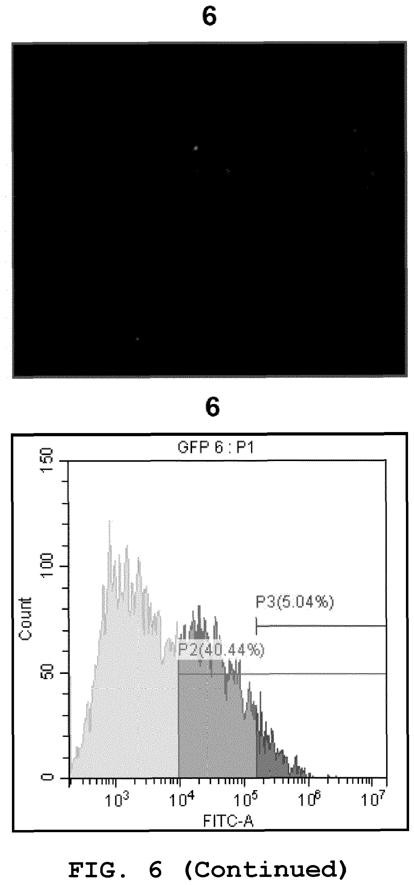

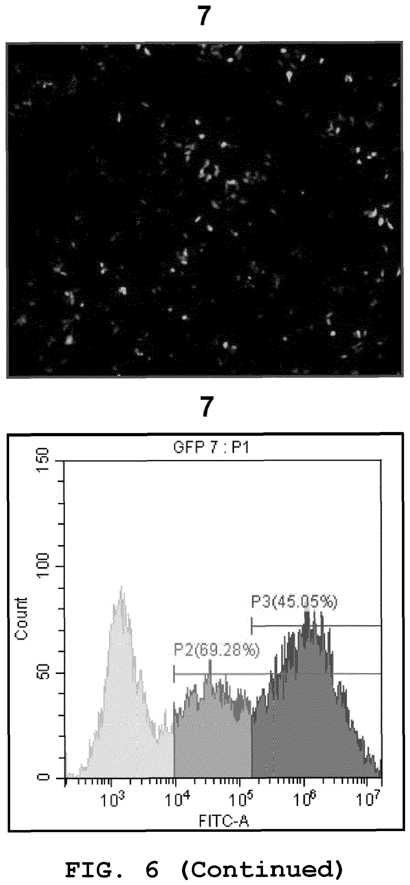

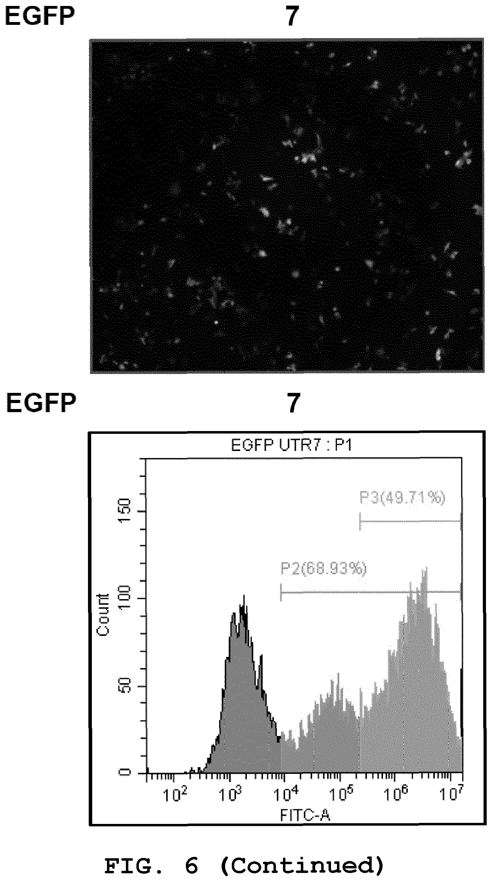

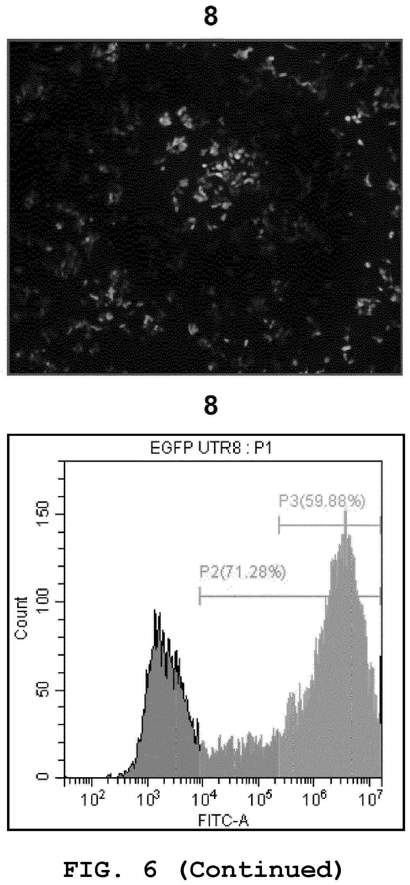

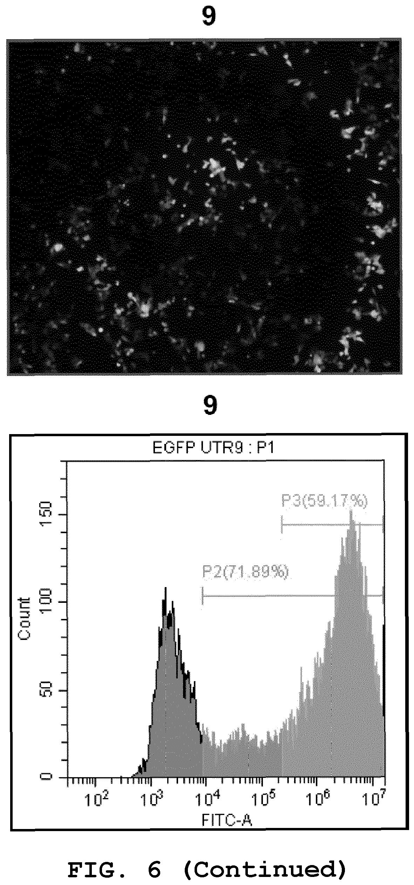

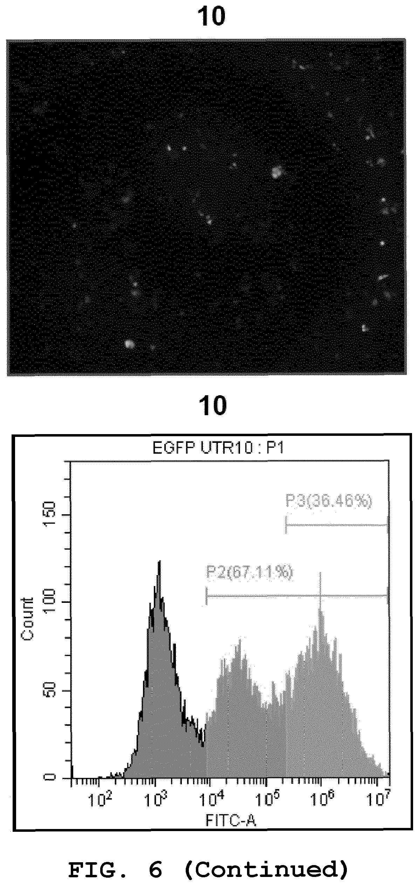

As a result of FACS analysis, the final expression form in the form of reduced UTR length was confirmed as UTR No. 9 form (see FIG. 6).

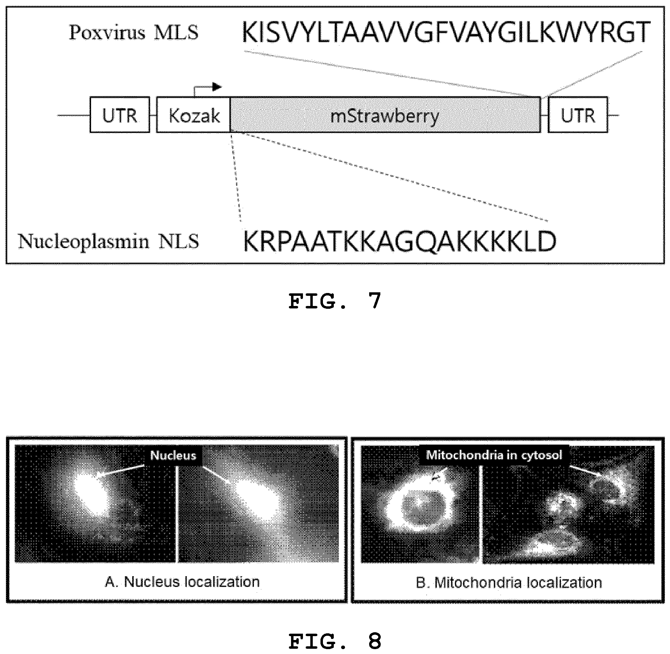

For diversification of the intracellular action sites based on UTR 9, in order to locate these sites in the nucleus, cytoplasm, and mitochondria where the nucleic acid synthesis pathway exists, the localization signal sequences (NLS: SEQ ID NO: 23, MLS: SEQ ID NO: 24) as shown in FIG. 7 were added, and the reporter gene mStrawberry was expressed according to the localization signal sequence, and the position of the protein was confirmed by fluorescences (see FIG. 8). The specific procedure of the fluorescent localization test (mRNA transfection: lipofectamine) according to the localization signal sequence is as follows.

One cover glass for confocal microscopy was put into each well of the 6-well cell culture plate, and 5 × 10⁵ cells were incubated in a cell incubator at 37°C and 5% CO₂ conditions for 24 hours to attach them to the cover glass. The mRNA to which each localization signal sequence and the mStrawberry reporter gene sequence expressing the red fluorescent protein are linked was transfected using lipofectamine messengerMAX reagent. First, 125 µL of OPTI-MEM medium and 3.5 µL of reagent in a micro-tube were mixed, incubated at room temperature for 10 minutes, and an mRNA dilution medium in another micro-tube, in which 125 µL of medium and 1.25 µg of reporter mRNA having each localization signal sequence were added and mixed, was added to the reagent and incubated for additional 5 minutes, and the resultant was administered to the cells of each well. After 4 hours, the cells of each well were slightly washed with phosphate buffered saline, replaced with a cell culture medium, and cultured for additional 20 hours to prepare a sample for observation under a confocal microscope. For sample preparation, 4% paraformaldehyde reagent was added thereto and incubated for 10 minutes to fix the cell, the resultant was washed with phosphate buffered saline, and 20 µL of a preservative solution was added on the slide glass, the fixed and washed cover glass was placed on it, and the adjacent area was cleaned without drying out, and it was confirmed whether the mStrawberry protein was located in the cell nucleus and the mitochondria such that the localization signal sequence was well operated with the red fluorescence of a confocal microscope.

T001 mRNA synthesis was performed as follows.

Template DNA preparation: The vector for *in vitro* transcribed (IVT) mRNA synthesis was modified in the pIRES vector. Briefly, the 5'UTR-T001-3'UTR cassette was cloned into the MCS of the pIRES vector. To prepare an IVT template, the plasmid was treated with *Sac*I/*Hpa*I enzymes to generate a linear strand, and the 1.5 kb linear strand containing the T7 promoter and T001 cassette was purely subjected to column purification and used as a template for PCR. The forward primer (gtgcttctgacacaacagtctcgaacttaagc; SEQ ID NO: 37) and the reverse primer (gaaGCGGCCGCCTTCCTACTCAGGCTTTATTC; SEQ ID NO: 38) were used in the PCR reaction, and all PCR reactions were performed using Pfu polymerase as follows: a total of 30 cycles at 95°C for 1 minute, at 61°C for 1 minute, and at 72°C for 3 minutes. PCR products were run on agarose gels and extracted using a Qiagen Cleanup Kit before further treatment.

IVT mRNA synthesis: After PCR, genetic information is transcribed from DNA to mRNA in vitro using the HiScribe^{™} T7 ARCA mRNA kit (New England Biolabs, Cat. #. E2065). 20 µL of an IVT reaction mixture was prepared by adding 10 µL of a NTP/cap analog mixture, 1 µg of template DNA, and 2 µL of 1× T7 RNA polymerase mixture to the reaction solution. The IVT reaction mixture was incubated at 37°C for 30 minutes. To remove the template DNA, 1 µL of DNase was added to the IVT reaction mixture and the mixture was incubated at 37°C for 15 minutes. For poly (A) tailing, 20 µL of the IVT reaction mixture was prepared by adding 5 µL of 10× poly (A) polymerase reaction buffer, 5 µL of poly (A) polymerase and 20 µL of nuclease-free water and the mixture was incubated at 37°C for 40 minutes. Thereafter, the resultant was purified using the RNeasy Mini Kit (Qiagen, Hilden, Germany) and the synthesized mRNA was purified by eluting on a spin column membrane with 89 µL of nuclease-free water. Then, the resultant was treated with 1 µL of Antarctic phosphatase at 37°C for 30 minutes to remove 5'-phosphate, purified again using the RNeasy Mini Kit (Qiagen, Hilden, Germany), and then eluted on a spin column with 50 µL of nuclease-free water to recover the synthesized mRNA. For the subsequent experiment, the synthesized mRNA was adjusted to a final concentration of 500 ng/uL, seeded, and stored at -80°C.

### Target metabolism in cancer cells

Thymidylate synthase (TS) is the only de *novo* source of thymidylate (dTMP) for DNA synthesis and repair. Drugs targeting the TS protein are the mainstay of cancer treatment, but their use is limited due to off-target effects and toxicity. Cytosolic thymidine kinase (TK1) and mitochondrial thymidine kinase (TK2) contribute to an alternative dTMP generation pathway by restoring thymidine from the tumor environment, and may modulate resistance to TS-targeting drugs. Since there was a report that downregulation of TKs with siRNA increased the capacity of TS siRNA to detect tumor cells compared to conventional TS protein targeting drugs (5FUdR and pemetrexed), the present invention was focused on dTTP biosynthesis and metabolism.

Although complex downregulation of these enzymes is an attractive strategy to enhance TS-targeted anticancer therapy, normal cytotoxicity and resistance to cancer drugs may be a problem. An alternative to avoid this defect is to obtain a highly dTMP-specific hydrolase, that is, T001. T001 can hydrolyze dTMP to thymidine without hydrolyzing other deoxynucleotide monophosphates (dNMPs). An imbalanced nucleotide pool in cells may be caused by dTTP deficiency and may lead to severe damage to cell growth and proliferation. Considering the substrate selectivity and activity of T001, the following hypotheses were proposed: (1) an unbalanced nucleotide pool may induce human tumor cells to accumulate damage, and (2) overexpression of T001 may cause an unbalanced nucleotide pool, and (3) an imbalanced nucleotide pool may cause a cell death by excessive repair frequency.

### Decrease in number of viable cells through T001 mRNAs transfection into cancer cells

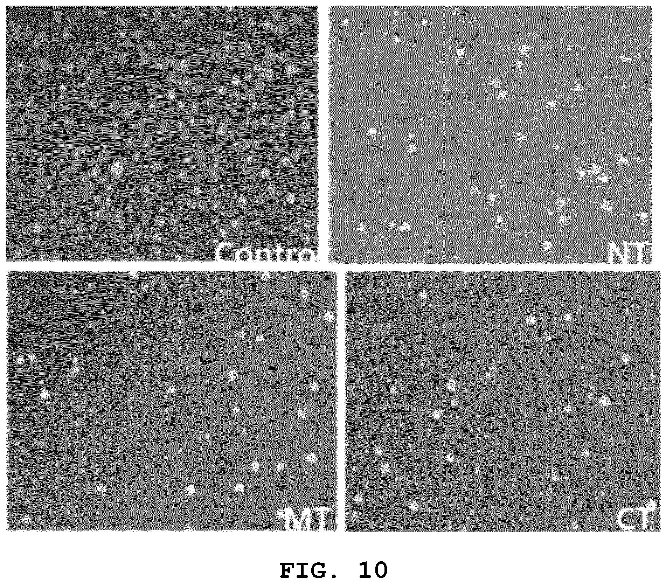

First, after transfecting each T001 mRNA, the number of cells in each group was estimated to evaluate tumor cell growth, and cell growth in culture was analyzed by a live and dead assay (see FIG. 10). Live and dead assays were performed as follows.

5 × 10⁵ cells were added into each well of a 6-well cell culture plate, and incubated for 24 hours in a cell incubator at 37°C and 5% CO₂ conditions to be attached to the bottom. After 24 hours, each mRNA version was transfected using lipofectamine and cultured for additional 24 hours. Cells from each experimental group were recovered, and the recovered cells were treated with 2 µM calcein AM and 4 µM EthD-1 (i.e., viability assay reagents), reacted for 30 minutes, and the cells were confirmed through a fluorescence microscope. Calcein AM enters the cell and displays green fluorescence after being degraded by the enzymes of a living cell, and EthD-1 enters the cell of a dead cell and stains the nucleus to show red fluorescence.

As shown in FIG. 10, the viable cells at 24 hours after transfection were significantly reduced compared to the control, and there was no difference between the mRNA versions (NT, MT, and CT).

### Inhibition of viable cell proliferation through T001 mRNAs transfection into cancer cells

MTT analysis was performed to quantify cell viability. The MTT analysis method is as follows.

10,000 cells were added per well in a 96-cell culture plate, and incubated for 24 hours in a cell incubator at 37°C 5% and CO₂ conditions to be attached to the bottom. After 24 hours, a lipofectamine reagent was prepared and transfected for each mRNA version and concentration, and cell activity assay experiments were performed for each time after incubation for additional 24 hours, 48 hours, and 72 hours. 10 µL of EZ-Cytox, which is i.e., a cell activity assay reagent, was administered to each sample per each well where cells were cultured for each time, and after reacting for 2 hours, the absorbance value at 450 nm wavelength was measured using a plate reader. Cell viability was analyzed per treatment time and concentration by comparing the values of the mRNA treatment group for each version compared to the control group.

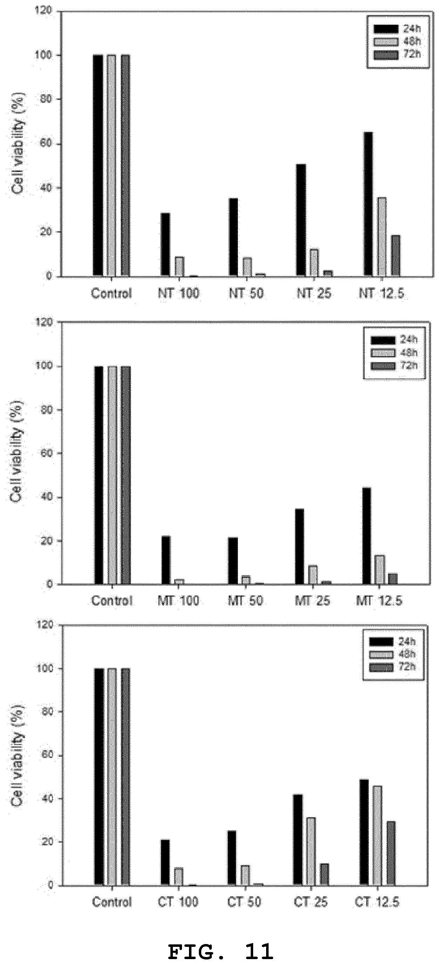

As a result of MTT analysis, compared to the control group, the proliferation of cells transfected with T001 mRNA was significantly inhibited 24 hours after transfection, and the inhibitory effect was maintained up to 72 hours, indicating that the viability of each cell was continuously reduced. Cell viability was shown to be different according to the dose (see FIG. 11).

### Induction of apoptosis through T001 mRNAs transfection into cancer cells

According to the experimental results, flow cytometric analysis was performed after Annexin V staining to examine whether T001 induces apoptosis to reduce cell number or inhibit cell proliferation. The specific analysis method is as follows.

5 ×10⁵ cells were added into each well of a 6-well cell culture plate, incubated for 24 hours in a cell incubator at 37°C and 5% CO₂ conditions to be attached to the bottom of the plate, and transfected with mRNA for each version using lipofectamine messengerMAX reagent. First, 125 µL of OPTI-MEM medium and 3.5 µL of the reagent were mixed in a micro-tube, and the mixture was incubated at room temperature for 10 minutes. In another micro-tube, an mRNA dilution medium in which 125 µL of medium and 1.25 µg of mRNA having each UTR structure are mixed, was added to the reagent above and incubated further for 5 minutes, and then administered to the cells of each well. After 4 hours, the cells in each well were slightly washed with phosphate buffered saline, replaced with a cell culture medium, and cultured further for 20 hours, detached from the plate bottom using trypsin enzyme, collected into a microcentrifuge tube, and then diluted in phosphate buffered saline. 3 µL of Annexin V staining reagent and Propidium Iodide staining reagent were added to each microcentrifuge tube, and after reaction at room temperature for 15 minutes, the degree of cell death induced by T001 version was compared through flow cytometry. As a control, mRNA-free lipofectamine messengerMAX reagent was used. Annexin V reagent stains the cell membrane of cells undergoing apoptosis, and Propidium Iodide stains the intracellular nucleus of dead cells. Therefore, on the flow cytometry graph, cells that do not undergo apoptosis are distributed in the third quadrant, and the position of the cell group moves from the fourth quadrant to the first quadrant according to the degree of apoptosis.

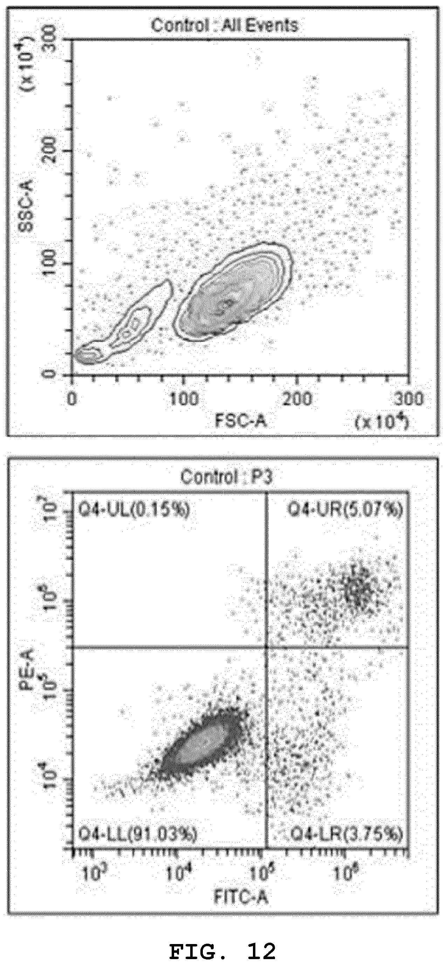

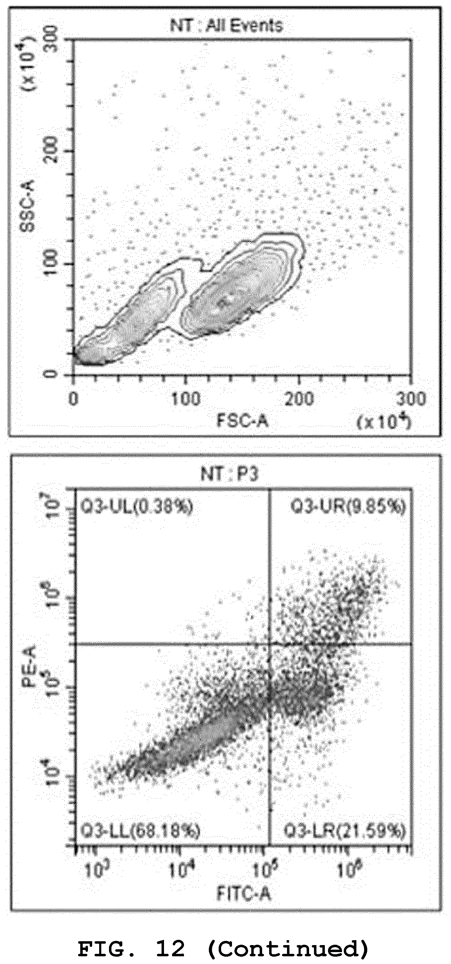

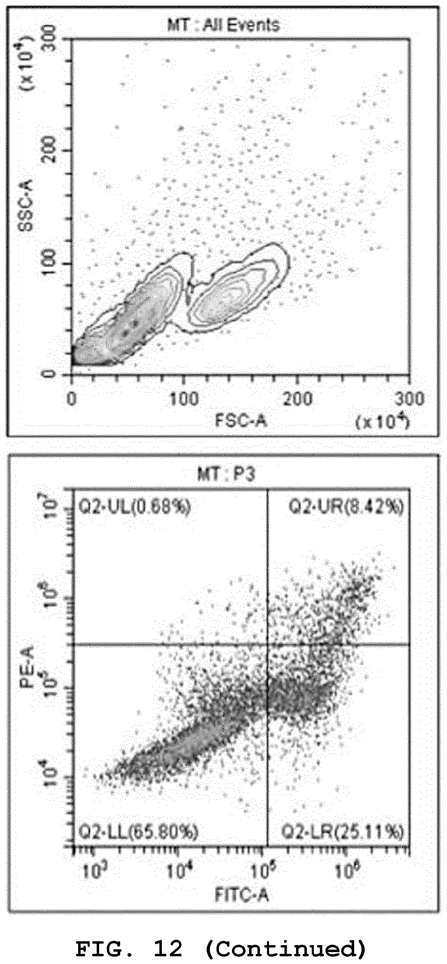

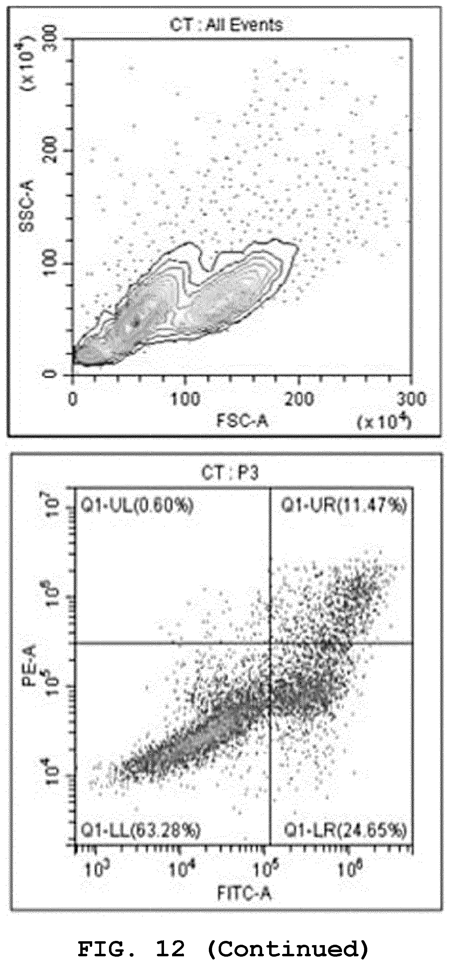

As a result of the analysis, apoptosis rates were similar in all versions. The apoptosis rate of the control group was 3.75%, and the premature death rates of cells transfected with NT, MT, and CT were 21.59%, 25.11%, and 24.65%, respectively. The apoptosis rates of cells metastasized with NT, MT, and CT were 9.85%, 8.42%, and 11.47%, respectively (see FIG. 12). The apoptosis rate increased in a dose-dependent manner showing some differences between each version of T001.

### Comparison of cytotoxicity of T001 and NT5M against colon cancer cells (HCT-116)

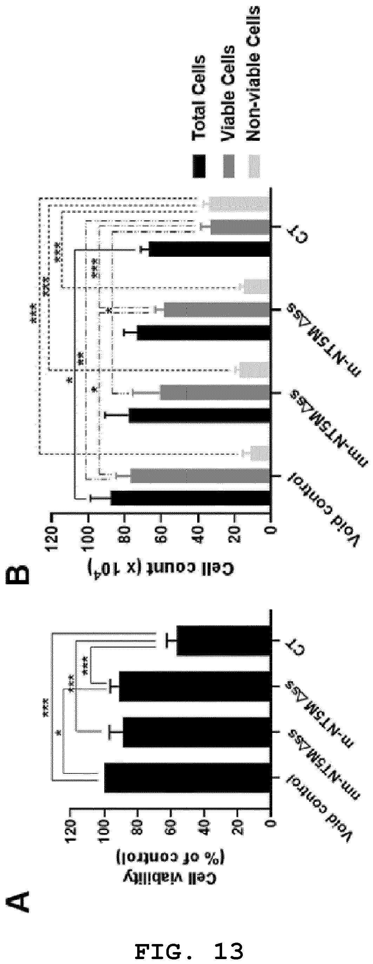

T001 is more specific to the nucleic acid T due to its structural difference from NT5M. Therefore, nucleic acid imbalance due to the loss of dTTP rather than the overall loss of nucleic acid may have a greater effect on cancer cell metabolism and thereby inhibit the growth of cancer cells. To prove this, a mature form of human NT5M and a non-mature form including introns were compared with cytoplasmic T001(CT) with respect to cell viability and cell growth inhibition effects between each enzyme so as to examine the effect on cells according to substrate specificity. Each mRNA was transfected into the colon cancer cell line HCT-116, and 24 hours thereafter, Trypan blue assay was performed and the results of cell viability and cell growth inhibition on HCT-116 are shown in FIG. 13. The specific experimental method is as follows.

HCT-116 cells were transfected with 1.25 µg each of non-mature form NT5M, mature form NT5M, and CT mRNA. After 24 hours, the resultants were each treated with trypsin and the cells were harvested using a centrifuge. After resuspending the harvested cells with 1× DPBS, a certain amount of cells were mixed with trypan blue reagent at a 1:1 ratio. After incubation for 2 minutes at room temperature (RT), the number of cells was counted using a hemocytometer. After obtaining the number of cells stained and unstained with the trypan blue reagent, the number of viable and non-viable cells was obtained, and then divided by the total number of cells to obtain the viability. Each experiment was repeated three or more times to evaluate the significance.

As shown in FIG. 13, in the case of T001 having high affinity and activity for dTMP, the effect on cells was greater than that of human NT5M with similar activity.

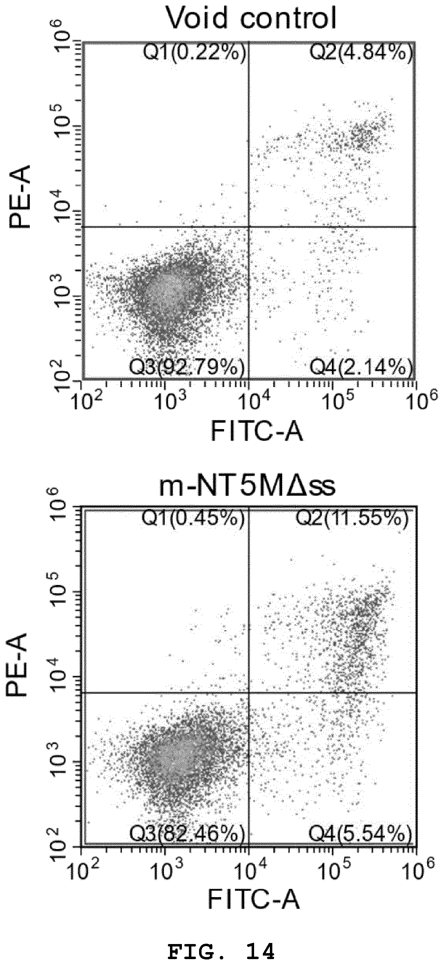

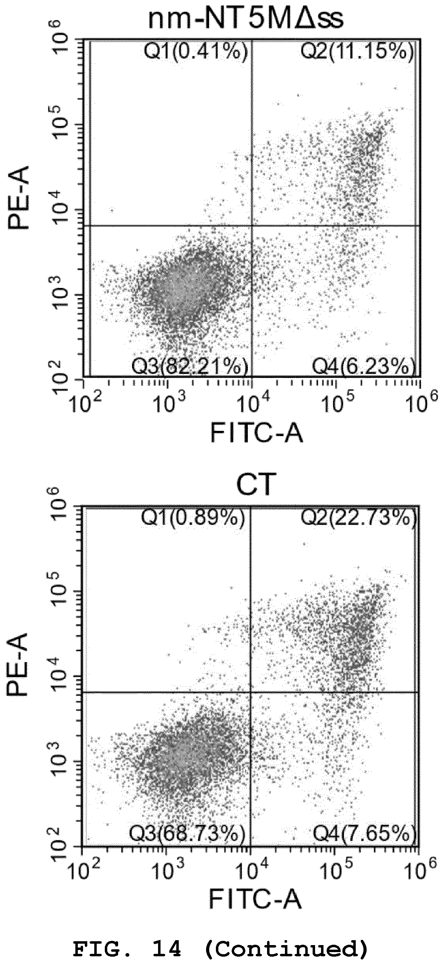

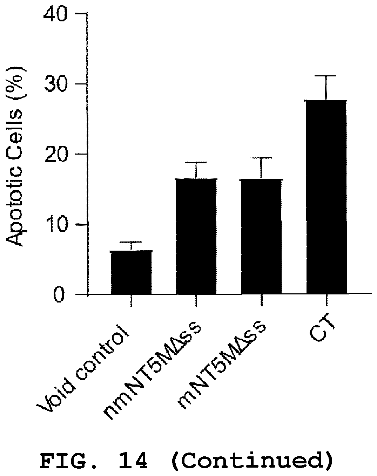

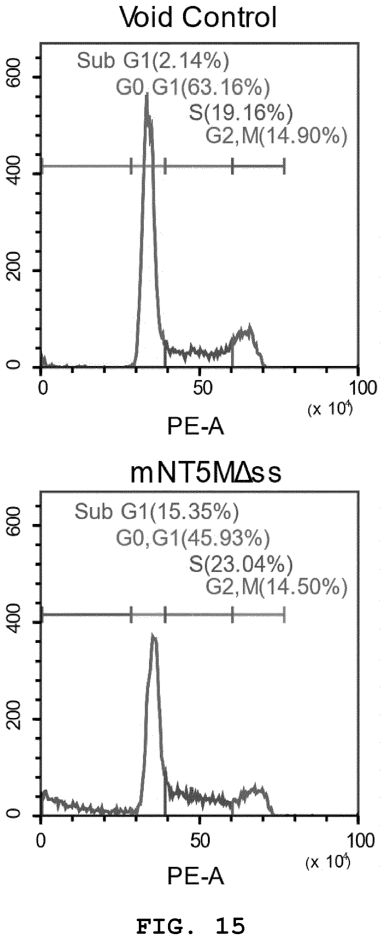

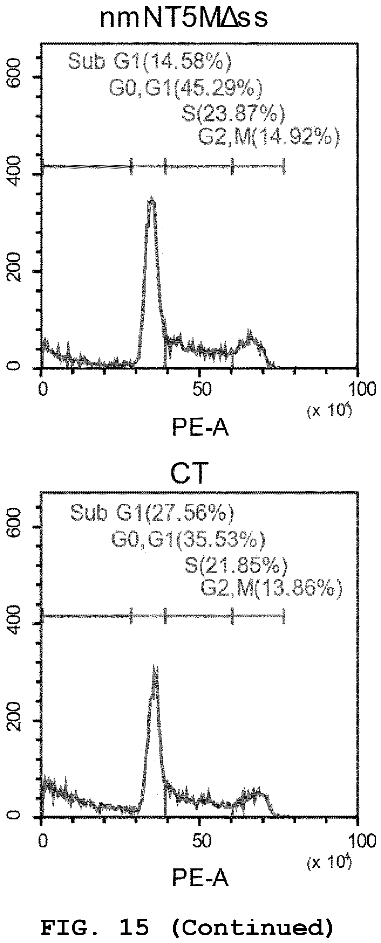

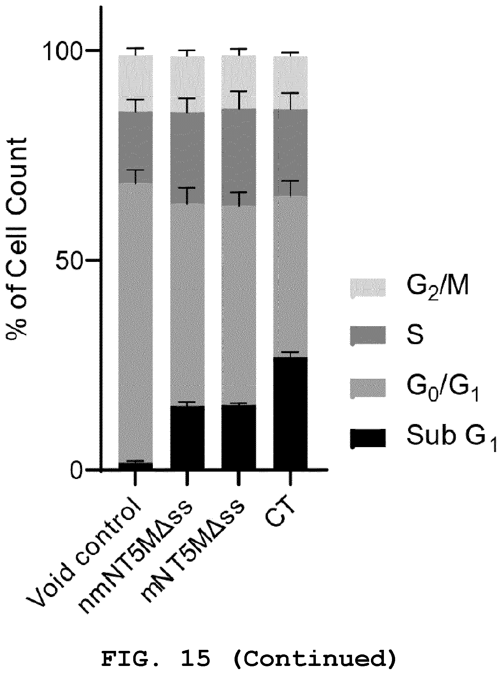

In particular, it was confirmed that the toxicity shown in the cells is mainly caused by the induction of apoptosis (see FIG. 14). Cells in which HCT-116 cells were transfected with immature NT5M, mature NT5M, and CT mRNA at 1.25 µg each were cultured for 24 hours and then the degree of apoptosis was measured and analyzed. As a result, while the two types of NT5M mRNA showed an increase of the Sub G1 group by about 12% compared to the control group, CT mRNA showed an increase by about 25% compared to the control group. The apoptosis caused by T001 expression is considered to be derived from the changes in cell cycle caused by intracellular nucleic acid deficiency, and it is predicted that when selective deficiency of specific nucleic acids and dTTP in the substrate causes cell cycle arrest and intensifies this process, it will eventually induce apoptosis (see FIG. 15).

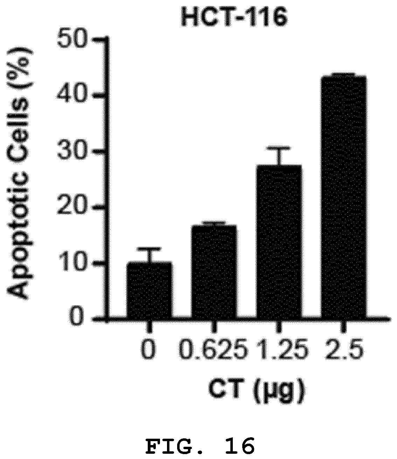

The apoptosis-inducing effect shown in the above results showed a concentration-dependent tendency according to the treatment concentration of CT in colon cancer cell lines, indicating that CT activity was directly involved in apoptosis (see FIG. 16).

In order to confirm whether the apoptosis of these cancer cells was an effect of T001, a change in the degree of apoptosis was confirmed after knocking down T001 using siRNA for CT. Two types of siRNA targeting T001 were prepared and transfected into HCT-116 cells, and then, CT mRNA was transfected. After 24 hours, the degree of apoptosis was confirmed through Annexin V/PI staining. The specific experimental method is as follows.

Two types of siRNA targeting T001 were prepared and transfected using RNAiMAX reagent, and CT mRNA was transfected 1 hour later. After 24 hours, cells were harvested and stained using the FITC Annexin V Apoptosis Detection Kit I. After incubation at room temperature for 20 minutes, the degree of fluorescence was analyzed using flow cytometry. 10,000 cells were analyzed per sample. The sequence of the siRNA used is as follows.
[siT001-1]
   Sense (SEQ ID NO: 33): CGAGAAGAAGUCAGAUUACAUCAAG
   Antisense (SEQ ID NO: 34): CUUGAUGUAAUCUGACUUCUUCUCG
[siT001-2]
   Sense (SEQ ID NO: 35): CGCAAAUUCAUUGAAACCUUCCUGA
   Antisense (SEQ ID NO: 36): UCAGGAAGGUUUCAAUGAAUUUGCG

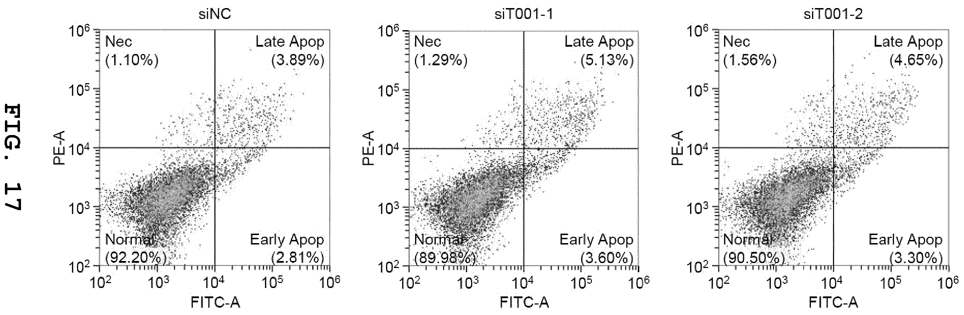

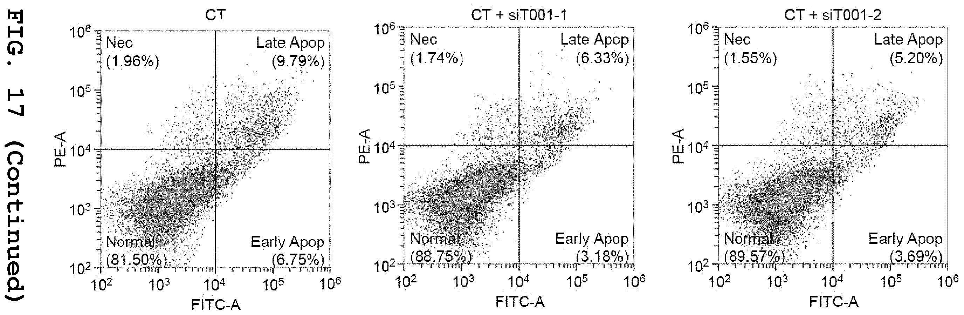

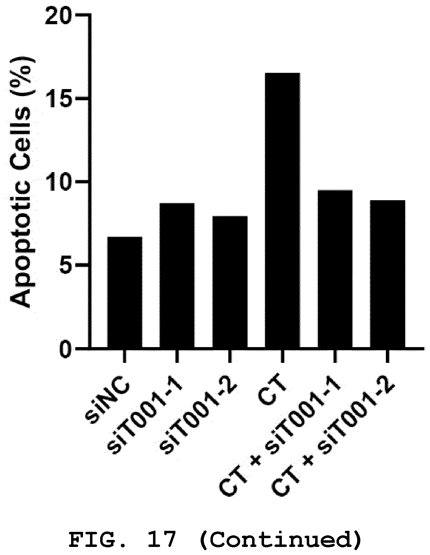

As a result of the analysis, it was confirmed that apoptosis was reduced in the group transfected with CT mRNA after transfection with siRNA compared to the group transfected with CT mRNA alone (see FIG. 17).

### Concentration-dependent inhibitory effect of T001 in triple-negative induced carcinoma

The mechanism of action of T001 was considered to be able to induce cancer cell death in carcinomas other than colon cancer, and thus, the same test was performed on triple-negative breast cancer (TNBC) to confirm the apoptosis-inducing ability. The specific test method is as follows.

MDA-MB-231 and MDA-MB-468 cells were each seeded at 5 × 10⁵ cells/well, and then transfected with 0 µg, 0.625 µg, 1.25 µg, and 2.5 µg of CT mRNA, respectively. After 24 hours, cells were harvested and stained using the FITC Annexin V Apoptosis Detection Kit I. After incubation at room temperature for 20 minutes, the degree of fluorescence was analyzed using a flow cytometer. 10,000 cells were analyzed per sample. Significance was evaluated through three repeated experiments.

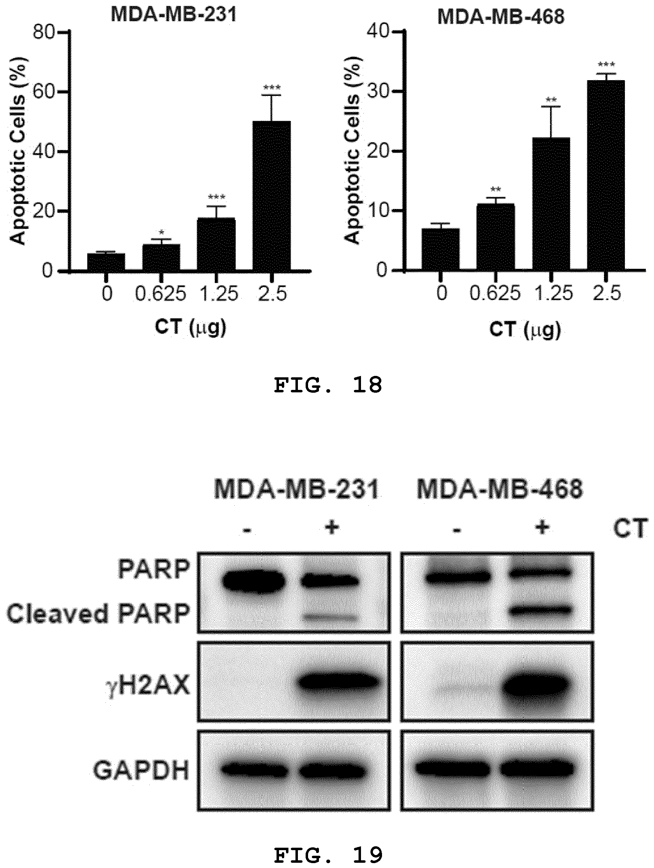

After treating two types of triple-negative breast cancer with cytoplasmic T001(CT) mRNA by concentration, cells cultured for 24 hours were subjected to FACS analysis through Annexin V/PI staining. As a result, it was confirmed that the ratio of apoptotic cells was also increased even in the carcinomas tested in proportion to the concentration of CT mRNA (see FIG. 18).

In order to analyze the protein level for the cause of apoptosis, 1.25 µg of CT mRNA was transfected into MDA-MB-231 and MDA-MB-468, which are TNBC cell lines, and then subjected to Western blot 18 hours thereafter. The specific analysis method is as follows.

After transfection with CT mRNA, cells were harvested 18 hours later. Cells were lysed on ice for 30 minutes using RIPA buffer (+protease inhibitor, phosphatase inhibitor), and then the protein was separated using a 4°C centrifuge for 15 minutes. The separated protein was quantified, and 10 µg to 20 µg of the protein was loaded onto an acrylamide gel. After the gel was transferred to the PVDF membrane, it was blocked with 5% skim milk at RT for 1 hour. Each 1' antibody was added thereto and the resultant was stored at 4°C overnight. After washing the resultant with 0.1% TBST, 2' antibody was added thereto and the resultant was incubated at RT for 1 hour. After washing the resultant with 0.1% TBST, a protein was detected using an ECL solution. A protein expression was analyzed using ChemiDoc XRS+.

As a result of the analysis, as shown in FIG. 19, the increase of expression of cleavage of PARP, which is an apoptosis marker and gamma-H2AX, which is a DNA damage marker. It was confirmed that DNA damage occurred due to the deficiency of dTTP during CT mRNA treatment, and it was confirmed that apoptosis was induced through the same.

### Anticancer agent using SV4 binder and T001 drug

Hereinafter, embodiments of anticancer agents using the SV4 binder and the T001 drug will be described.

Anticancer agents using SV4 binder and T001 drug are fourth-generation targeted anticancer agents, which target the immune evasion and metabolic vulnerability of cancer cells and thereby remarkably reduce side effects of normal cells, in such a manner that they primarily detect and bind to CD47 overexpressed on the surface of cancer cells, and secondarily, mRNA-type nucleic acid metabolism inhibitors that enter cancer cells detect and inhibit excessive nucleic acid synthesis metabolism of cancer cells. In general, cancer cell-specific surface proteins are often distributed in normal cells, and thus damage to normal cells is inevitable when a target protein capable of recognizing a specific surface protein is coupled to a toxic material. However, recognition alone does not produce toxicity, and if the metabolism of cancer cells that excessively attempts nucleic acid synthesis is targeted, the recognition rate for cancer cells becomes high, thereby reducing damage to normal cells. That is, most normal cells that recognize CD47 and do not amplify nucleic acid for growth even if T001 mRNA is introduced into the cells would have little damage because the demand for nucleic acid is small, but cancer cells that can grow only by synthesizing excessive nucleic acid would have significant damage due to imbalance of nucleic acid caused by deficiency of dTMP. Therefore, even if some normal cells with a relatively high CD47 level are targeted in the process of recognizing cancer cells with high expression of CD47, damage to normal cells can be reduced by increasing the recognition rate of cancer cells by causing greater damage to the cancer cells through the targeting nucleic acid metabolism introduced into the inside.

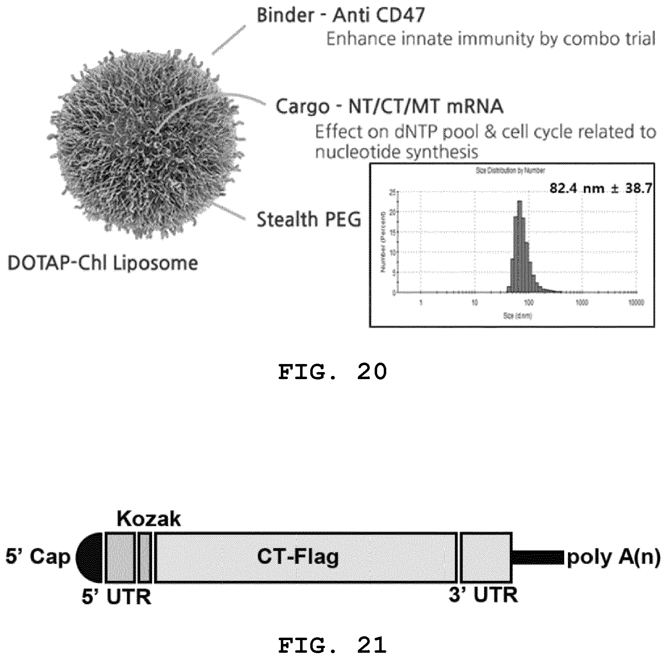

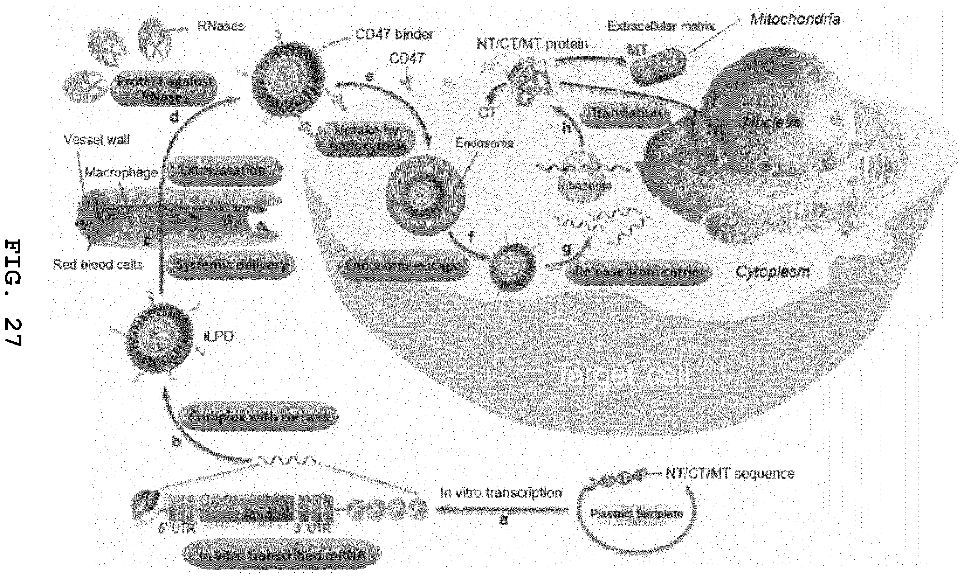

As shown in FIG. 20, the constitution of the anticancer agent using the SV4 binder is in the form of a liposomal nanoparticle, in which it has a CD47 recognition protein on the outside and mRNAs, in which localization signals (intranuclear, intracytoplasmic, and intramitochondrial) were in a different combination of the cancer types, are captured inside. FIG. 27 schematically shows a mechanism of the anticancer agent using the SV4 binder according to the present invention.

### Components of anticancer agent

### (1) CD47 binder (SV4)

In conjugating DSPE to a high-affinity CD47 conjugate, which is a modification of a Sirpα-derived protein, SV4, which was modified to be located outside the liposome in the correct orientation, was used as a CD47 binder, and the predicted structure of SV4 is shown in FIG. 1A. As a result of comparing the K_{D} values of the sample bound to DSPE and the sample in which DSPE was inserted into the liposome, it can be seen that the liposome form was improved by sequence mutation (see Table 1 above).

### (2) Liposome-based drug delivery system constituents

As materials used to positively charge the liposome to achieve smooth drug delivery to the target, the structures of cationic phospholipid (DSPE-PEG₁₀₀₀), DOTAP, and cholesterol are shown in Formulas 1 to 3, respectively.

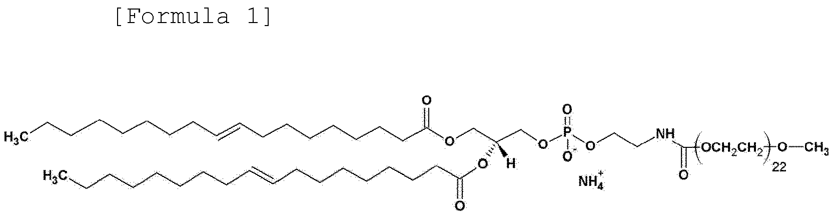

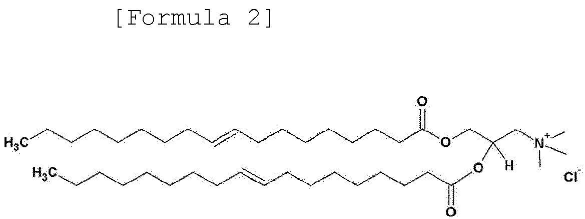

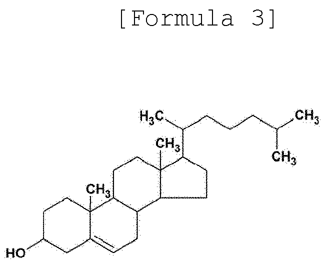

### (3) Drugs

As described above, the structure of T001 mRNA is schematically shown in FIG. 21 as mRNA capable of intranuclear, intracytoplasmic, and intramitochondrial expression.

### Positioning of translated proteins by fused mStrawberry mRNA with localization signals

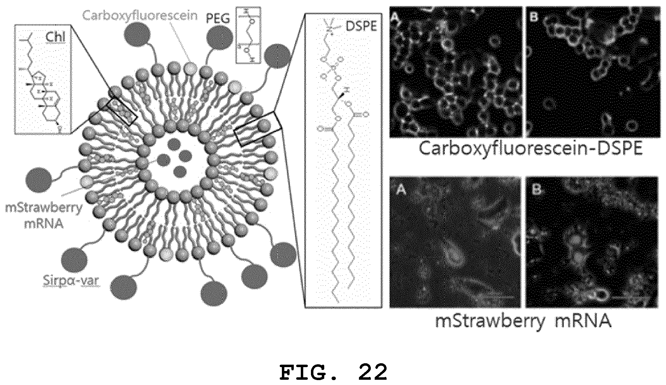

In order to confirm whether localization signals worked well, mRNA was transfected into MCF7 with nuclear and mitochondrial localization signals. mStrawberry fluorescence was detected to confirm successful transfection and localization of each mRNA (see FIG. 22). The specific experimental method is as follows.

5 × 10⁵ cells were added into each well of a 6-well cell culture plate, and incubated for 24 hours in a cell incubator at 37°C and 5% CO₂ conditions to be attached to the bottom of the plate. mStrawberry mRNA producing a red fluorescent protein linked to an intracellular cytoplasmic localization signal synthesized in the laboratory was captured in a liposome constructed using carboxyfluorescein-conjugated DSPE, and transfected by treating with MCF-7 cells whose 24-hour culture was completed. After additional 24 hours of incubation, the location of carboxyfluorescein of liposomes was confirmed on green fluorescence cells with green wavelength under a confocal laser fluorescence microscope, and it was confirmed that mRNA transfected with red wavelength produced mStrawberry protein and was located in the cytoplasm.

### Confirmation of CD47-mediated drug delivery

In order to confirm whether drug delivery is mediated by CD47, MCF-7 cells were treated with SV4-conjugated iLP where the drug epirubicin was captured. In this case, an experimental group treated with the CD47 antibody (polyclonal) and a non-treated experimental group were compared to determine whether the drug delivery was mediated by CD47. The specific CD47 masking assay method is as follows.

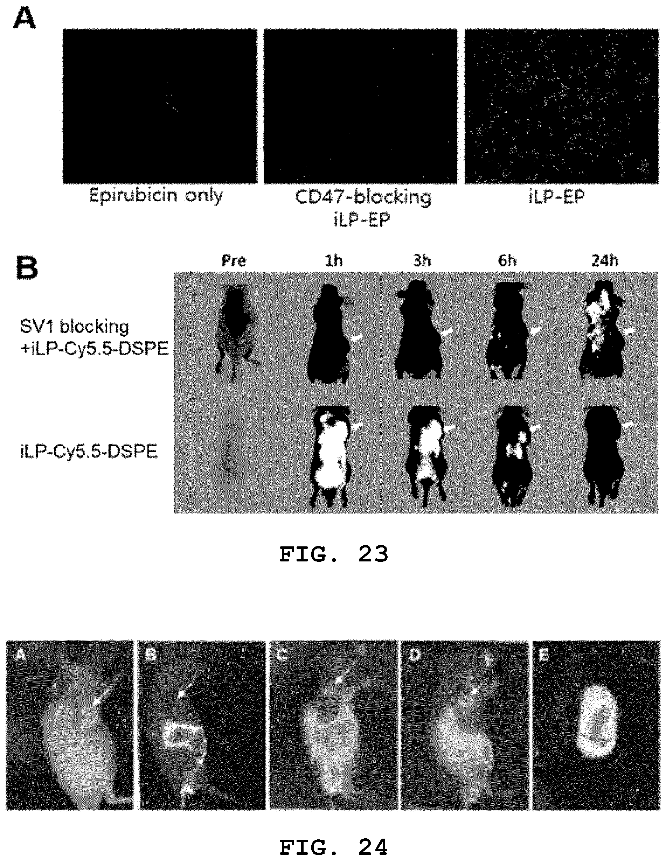

5 × 10⁵ Cells were added into each well of a 6-well cell culture plate, and incubated for 24 hours in a cell incubator at 37°C and 5% CO₂ conditions to be attached to the bottom. CD47 antibody was added to only one cell sample to a final concentration of 5 ug/mL and reacted for 4 hours to block CD47 on the cell surface. Epirubicin was captured therein and each cell was treated with SV4-conjugated liposomes (iLP) to a final epirubicin concentration of 10 µM and cultured for 24 hours. After 24 hours, the fluorescence level of epirubicin, which was introduced into the cell and exhibited red fluorescence in the cell nucleus, was comparatively analyzed through a fluorescence microscope. In this case, a cell sample directly treated with epirubicin was used as a control (see FIG. 23A). 100 µg of SV4 protein was first administered to MCF7-xenograft mice and saturated to prepare a state in which CD47 of cancer cells in the body of the mouse is blocked, and the same volume of phosphate buffered saline was administered to prepare a state in which CD47 of cancer cells is unblocked. Here, 100 µL (5 µg of mRNA/0.5 mg of liposome) of liposomes, in which SV4-DSPE and Cyanine 5.5-DSPE exhibiting red fluorescence in the near-infrared region were simultaneously inserted, was administered into the body of the mouse through intravascular injection to confirm the CD47-mediated accessibility to the target.

As a result of the experiment, as shown in FIG. 23A, epirubicin fluorescence was not observed in the treatment group where CD47 was blocked, but epirubicin fluorescence was observed in the experimental group where CD47 was unblocked.

Additionally, in the MCF7-xenograft mouse model experiment to confirm the CD47-mediated drug delivery ability *in vivo,* overall noise was observed due to the CD47 present in blood cells; however, stronger drug delivery was confirmed in the cancer cells of mice where CD47 was unblocked compared to mice blocked with SV4 (see FIG. 23B).

In the MCF7 xenograft mouse model, SV4-conjugated iLP containing the mRNA of Near Infrared Red Fluorescence Protein (NIR RFP) was injected intravenously, and then the fluorescence images were analyzed by time. The results are shown in FIG. 24. The specific experimental method is as follows.

MCF7, a human breast cancer cell line, was cultured and xenografted into nude mice. SV4-iLP, in which NIR-RFP (Near Infrared-Red Fluorescence Protein) mRNA was captured, was administered by adjustment through intravenous injection so that 5 µg mRNA per 25 g mouse could be administered, and then, the fluorescence level of the sample injected using an *in vivo* optical imaging system was tracked.

Referring to FIG. 24, a phenomenon was observed in which although mRNA delivery occurs through CD47 of some blood cells, expression is accumulated in cancer cells at a relatively high level.

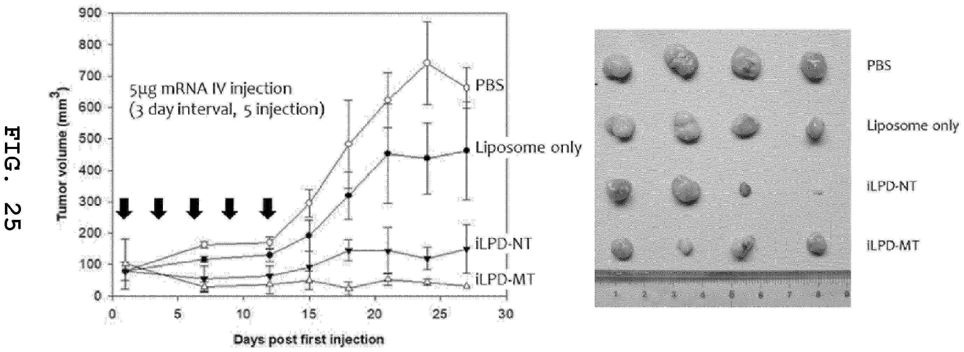

Cancer growth control efficacy test (28 days) was performed in an MCF7 xenograft mice IV, and specifically, MCF7 was cultured and subcutaneously injected into nude mice. After randomly classifying nude mice with a grown tumor, the mice were treated with PBS, liposome, liposome-NT, and liposome-MT (20 mpk of liposome, 10 µg mRNA/mg of liposome) were treated intravenously (IV), respectively. Each mRNA was treated in an amount 5 µg, and a total of 5 treatments were performed for 28 days at 3day intervals. The tumor volume was examined at 3 day intervals and the results are presented as a graph. On the 28th day, after sacrificing the nude mice, the tumors were isolated and their volumes were measured. The results are shown in FIG. 25.

Referring to FIG. 25, tumor growth rates were remarkably reduced in the iLPD-NT and iLPD-MT groups compared to the two control groups (P = 0.03). Additionally, there were some differences between the PBS and void liposome-injected samples.

In order to evaluate the toxic effect of mRNA *in vivo,* the body weight of mice was measured during the experiment. As a result, it was found that the body weight of mice in all groups increased slightly over the entire experimental period, and the change in body weight after sample injection was not significant compared to the control group. As shown in FIG. 25, the final size of the tumor tissue collected from the mice sacrificed after completion of the experiment was compared and the results are presented.

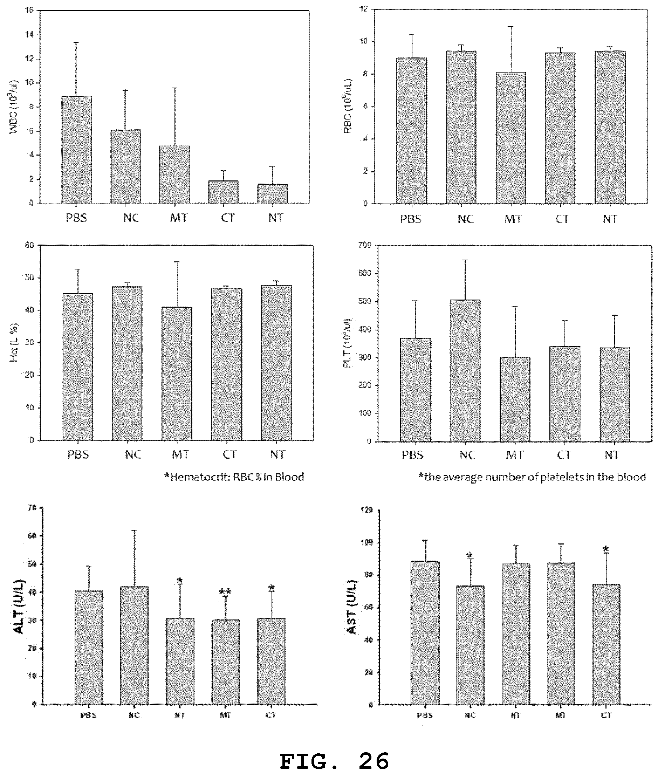

Hematological and histological examinations were performed with blood and tissues collected from the mice sacrificed after completion of the experiment, and the results are shown in FIG. 26. Referring to FIG. 26, there was hardly any significant toxic effect except for the number of WBCs in the blood and liver of the mice treated with the sample, compared to the control group.

Preferred embodiments of the present invention described above are disclosed to solve the technical tasks, and various modifications, changes, additions, etc. will be possible within the spirit and scope of the present invention by those of ordinary skill in the art to which the present invention pertains, and such modifications and changes should be regarded as belonging to the following claims.

## Claims

1. A binder which binds to CD47 overexpressed in cancer cells, comprising an amino acid sequence represented by SEQ ID NO: 1 or SEQ ID NO: 2.

2. The binder of claim 1, wherein the binder is 1,2-distearoyl-sn-glycero-3-phosphorylethanolamine (DSPE)-conjugated.

3. The binder of claim 1, wherein the cancer is colon cancer or breast cancer.

4. A binder-conjugated liposome complex, wherein the binder of claim 1 is conjugated to a liposome.

5. The binder-conjugated liposome complex of claim 4, wherein the liposome is positively charged using at least one material selected from the group consisting of cationic phospholipid, DOTAP, and cholesterol.

6. The binder-conjugated liposome complex of claim 4, wherein the liposome is PEGylated.
